# EUROPÄISCHE PATENTSCHRIFT

(11) **EP 1 778 176 B1**
(45) Veröffentlichungstag und Bekanntmachung des Hinweises auf die Patenterteilung: **25.07.2012**
(21) Anmeldenummer: 05746210.3
(22) Anmeldetag: 27.05.2005
(51) Int. Cl.: A61K 8/49, A61Q 5/10

(54) **MITTEL ZUM FÄRBEN VON KERATINHALTIGEN FASERN**
COMPOSITION FOR COLOURING KERATIN-CONTAINING FIBRES
COMPOSITION POUR LA COLORATION DE FIBRES KÉRATINIQUES

(30) Priorität: 12.06.2004 DE 102004028598
(43) Veröffentlichungstag der Anmeldung: 02.05.2007
(73) Patentinhaber: Henkel AG & Co. KGaA, 40589 Düsseldorf (DE)
(72) Erfinder: SÜNGER, Georg, 22081 Hamburg (DE); GROSS, Wibke, 40549 Düsseldorf (DE); HÖFFKES, Horst, 40595 Düsseldorf (DE); OBERKOBUSCH, Doris, 40591 Düsseldorf (DE); BENICKE, Wolfgang, 40723 Hilden (DE)
(86) Internationale Anmeldenummer: PCT/EP2005/005740
(87) Internationale Veröffentlichungsnummer: WO 2005/120445

(56) Entgegenhaltungen:
- EP-A- 1 407 756
- DE-A1- 2 357 442
- DE-A1- 2 446 632
- DE-A1- 4 107 439
- US-A- 2 473 166
- US-A- 4 256 896
- US-A- 4 992 204

## Beschreibung

Die Erfindung betrifft ein Mittel zum Färben von keratinhaltigen Fasern, insbesondere menschlichen Haaren, das ausgewählte Acetonitrilderivate in Kombination mit reaktiven Carbonylverbindungen enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterozyklische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridin-derivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Problematisch gestaltet sich nach wie vor eine Bereitstellung von Oxidationshaarfärbungen mit ausreichenden Echtheitseigenschaften, insbesondere in mit sehr guten Wasch- und Reibechtheiten.

Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel, enthaltend CH-acide Acetonitrilderivate gemäß nachstehender Formel I in Kombination mit reaktiven Carbonylverbindungen, sowie der Verwendung dieser Kombination zum Färben von keratinhaltigen Fasern oder zur Farbauffrischung bzw. Nuancierung von bereits gefärbten keratinhaltigen Fasern sind bislang nicht bekannt.

In der deutschen Offenlegungsschrift 24 46 632 werden Haarfärbemittel offenbart, die als Acetonitrilderivate aromatische bzw. heteroaromatische Cyanacetylverbindungen in Kombination mit Oxidationsfarbstoffvorprodukten. Kombinationen dieser Cyanacetylverbindungen mit reaktiven Carbonylverbindungen werden nicht beschrieben.

Aufgabe der vorliegenden Erfindung ist es, Färbemittel für keratinhaltige Fasern, insbesondere menschliche Haare, bereitzustellen, die hinsichtlich der Farbtiefe, der Grauabdeckung und den Echtheitseigenschaften, wie beispielsweise Licht-, Reib- und Waschechtheit sowie Schweiß- und Kaltwellechtheit, qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten Verbindungen, insbesondere in Kombination mit Verbindungen enthaltend mindestens eine reaktive Carbonylgruppe, sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden insbesondere Ausfärbungen mit verbesserten Echtheitseigenschaften über einen Nuancenbereich von gelb über gelbbraun, orange, braunorange, braun, rot, rotviolett bis hin zu blauviolett und schwarz erhalten. Der Einsatz von oxidierenden Agentien soll jedoch nicht prinzipiell ausgeschlossen werden.

Gegenstand der Erfindung ist ein Mittel zum Färben von keratinhaltigen Fasern, insbesondere menschlichen Haaren, enthaltend in einem wasserhaltigen kosmetischen Träger als Komponente A mindestens eine Verbindung gemäß Formel I oder deren physiologisch verträgliches Salz, 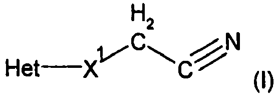 worin
● Het steht für einen gegebenenfalls substituierten Heteroaromaten,
● X¹ steht für eine direkte Bindung oder eine Carbonylgruppe,
und als Komponente B mindestens eine Verbindung mit einer reaktiven Carbonylgruppe.

Der Rest Het gemäß Formel I steht bevorzugt für das Molekülfragment mit der Formel II, 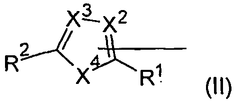 worin
● R¹ und R² stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{I}R^{II}N-(CH₂)ₘ-, worin R¹ und R^{II} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{I} und R^{II} gemeinsam mit dem Stickstoffatom einen 5-, 6- oder 7-gliedrigen Ring bilden können und m steht für eine Zahl 0, 1, 2, 3 oder 4, wobei R¹ und/oder R² einen an den Ring des Restmoleküls ankondensierten, gegebenenfalls substituierten aromatischen oder heteroaromatischen, 5- oder 6-Ring bilden können
● X² und X³ stehen unabhängig voneinander für ein Stickstoffatom oder eine Gruppe CR³, wobei R³ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{III}R^{IV}N-(CH₂)ₙ-, worin R^{III} und R^{IV} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{III} und R^{IV} gemeinsam mit dem Stickstoffatom einen 5-, 6- oder 7-gliedrigen Ring bilden können und n steht für eine Zahl 0, 1, 2, 3 oder 4, wobei mindestens einer der Substituenten X² und X³ zusammen mit dem Restmolekül einen ankondensierten gegebenenfalls substituierten aromatischen 5-oder 6-Ring bilden kann,
● X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylgruppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{V}R^{VI}N-(CH₂)ₚ- steht, worin R^{V} und R^{VI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{V} und R^{VI} gemeinsam mit dem Stickstoffatom einen 5-, 6- oder 7-gliedrigen Ring bilden können und p steht für eine Zahl 0, 1, 2, 3 oder 4, substituiert sein können,
mit der Maßgabe, daß, wenn X⁴ für eine Vinylengruppe steht, mindestens eine der Gruppen X² oder X³ ein Stickstoffatom bedeutet.

Die Bindung des heterozyklischen Rings gemäß Formel II zum Molekülfragment -X¹-CH₂-C≡N unter Erhalt der erfindungsgemäßen Verbidung gemäß Formel I erfolgt an den Ring des Heterozyklusses und ersetzt ein an diesen Ring gebundenes Wasserstoffatom. Folglich ist es zwingend notwendig, daß die Substituenten R¹, R², X², X³ und X⁴ derart gewählt werden müssen, daß mindestens einer dieser Substituenten eine entsprechende Bindungsbildung ermöglicht. Folglich ist es zwingend, daß mindestens einer der Reste R¹ oder R² die Bindung zum Molekülfragment -X¹-CH₂-C≡N ausbildet, wenn X⁴ ein Sauerstoffatom oder ein Schwefelatom ist und X² und X³ ein Stickstoffatom bedeuten.

Der Rest Het gemäß Formel I wird besonders bevorzugt abgeleitet von den Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hadroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise sind die Verbindungen gemäß Formel I ausgewählt aus der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril.

Im folgenden sollen Beispiele für die als Substituenten im Rahmen dieser Anmeldung genannten Gruppen bzw. Reste erwähnt werden. Beispiele für C₁-C₆-Alkylgruppen sind die Gruppen Methyl, Ethyl, Propyl, Isopropyl, n-Butyl, sec-Butyl und tert.-Butyl, n-Pentyl und n-Hexyl. Propyl, Ethyl und Methyl sind bevorzugte Alkylgruppen. Beispiele für entsprechende zyklische Alkylgruppen sind Cyclopentyl und Cyclohexyl. Beispiele für bevorzugte C₂-C₆-Alkenylgruppen sind Vinyl und 2-Propenyl (Allyl). Erfindungsgemäß bevorzugte C₁-C₆-Alkoxygruppen sind beispielsweise eine Methoxy- oder eine Ethoxygruppe. Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für C₁-C₆-Alkoxycarbonylgruppen; die Methoxycarbonyl-, die Ethoxycarbonylgruppe und die tert-Butoxycarbonylgruppe sind dabei besonders bevorzugt. Weiterhin können als bevorzugte Beispiele für eine C₁-C₆-Hydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt. Die Methoxyethyl-, Ethoxyethyl-, Methoxypropyl-, Methoxybutyl-, Ethoxybutyl- und die Methoxyhexylgruppe sind Beispiele für erfindungsgemäße C₁-C₆-Alkoxy-C₂-C₆-alkylgruppen. Beispiele für eine C₂-C₆-Polyhydroxyalkylgruppe sind die 2,3-Dihydroxypropylgruppe, 3,4-Dihydroxybutylgruppe und die 2,4-Dihydroxybutylgruppe. Eine bevorzugte Hydroxy-C₁-C₆-alkoxygruppe ist die 2-Hydroxyethoxygruppe. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Beispiele für eine Heterozyklus-(C₁-C₄)-alkylgruppe sind Pyrrolidino-(C₁-C₄)-alkyl, Piperidino-(C₁-C₄)-alkyl, Morpholino-(C₁-C₄)-alkyl, 2-Furyl-(C₁-C₄)-alkyl, 2-Thienyl-(C₁-C₄)-alkyl, 4-Pyridyl-(C₁-C₄)-alkyl, 3-Pyridyl-(C₁-C₄)-alkyl, 2-Pyridyl-(C₁-C₄)-alkyl, Triazolyl-(C₁-C₄)-alkyl und 1-Imidazolyl-(C₁-C₄)-alkyl. Die Arylgruppen und die Heteroarylgruppen sind gegebenenfalls substituiert bevorzugt mit einer oder mehreren Gruppen, die ausgewählt werden aus einem Halogenatom, einer Hydroxygruppe, einer Aminogruppe, einer C₁-C₆-Alkoxygruppe, einer C₁-C₆-Alkylgruppe, einer Nitrogruppe, einer Carboxygruppe, einer Carboxamidogruppe und einer Cyanogruppe. Beispiele für Halogenatome sind F-, Cl-, Br- oder I-Atome, wobei Cl-Atome ganz besonders bevorzugt sind. Bevorzugte C₁-C₆-Aminoalkylgruppen sind die Aminomethyl-, die Aminoethyl und die Aminopropylgruppe. Bevorzugte Aryl-C₁-C₆-alkylgruppen sind Benzyl und 2-Phenylethyl. Die Aminomethyl-, 2-Aminoethyl-, 3-Aminopropyl-, 2-Dimethylaminoethyl-, Diethylaminomethyl-, Dimethylaminomethyl, 2-Methylaminoethyl-, Dimethylamino-, Piperidinomethyl-, Pyrrolidinomethyl, Morpholinomethyl- und die Aminogruppe sind Beispiele für eine Gruppe R'R"N-(CH₂)ₙ-, wobei die Diethylaminomethyl-, Piperidinomethyl, 2-Dimethylaminoethyl-, Dimethylamino- und die Aminogruppe besonders bevorzugt sind. Eine bevorzugte C₁-C₆-Carboxyalkylgruppe ist die 3-Carboxypropylgruppe. Besonders bevorzugte C₂-C₆-Alkenylengruppen sind Vinylen und Propylen. Eine besonders bevorzugte C₄-C₆-Alkadienylengruppe ist die 1,3-Butadien-1,4-diylgruppe. Die Gruppen 1-Carboxypropylen und 1-Carboxyethylen sind bevorzugte Carboxy-(C₁-C₄)-alkylengruppen. Die weiteren verwendeten Begriffe leiten sich erfindungsgemäß von den hier gegebenen Definitionen ab.

Die erfindungsgemäßen Verbindungen gemäß Formel I sind CH-acide Verbindungen. Sie können in alkalischer Lösung am Kohlenstoffatom in Nachbarschaft zur Gruppe ―CN zu einer aktiven Methylenkomponente deprotoniert werden. Letztere reagiert mit der reaktiven Carbonylverbindung unter Ausbildung eines chromophoren Systems.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden.

Verbindungen der Formel I sind nach bekannten Syntheseverfahren gemäß W. S. Emerson et al., J. Org. Chem., 1948, 13, 722-728; S. A. Lang et al., J. Med. Chem., 1975, 18(4), 441-443, K. Jänisch et al., Journal f. prakt. Chemie, 1987, 329, 171-175, O. Hromatka et al., Monatshefte für Chemie, 1976, 107, 235-239, R. Kreher et al., Chem. Ber., 1980, 113, 3675-3677, K. Rehse et al., Arch. Pharm. (Weinheim), 1978, 311, 52-58, P. H. J. Ooms et al., Synthesis, 1975, 10, 639-641, S. Laufer et al., Arch. Pharm. (Weinheim), 1997, 330, 307-312, F. Eiden et al., Arch. Pharm. (Weinheim), 1985, 318(12), 1096-1100, D. N. Ridge et al., J. Med. Chem., 1979, 22(11), 1385-1389, E. Ohsugi et al., Chemical & Pharmaceutical Bulletin, 1989, 37(5), 1268-1278 und P. Angiuli et al., Journal of Labelled Compounds and Radiopharmaceuticals, 1990, 28(10), 1213-1219 herstellbar. Alle in diesen Literaturvorschriften nicht erwähnten Verbindungen der Formel I können in Analogie zu den Synthesevorschriften dieser Literaturstellen dargestellt werden.

Färbungen mit erhöhter Brillanz und verbesserten Echtheitseigenschaften (z.B. Waschechtheit, Reibechtheit und Schweißechtheit) über einen weiten Nuancenbereich werden erzielt, wenn die eingesetzten Verbindungen der Formel I erfindungsgemäß gemeinsam mit mindestens einer Substanz mit einer reaktiven Carbonylgruppe (im folgenden auch Komponente B oder reaktive Carbonylverbindung genannt) in den erfindungsgemäßen Mitteln enthalten sind. Erfindungsgemäße reaktive Carbonylverbindungen besitzen mindestens eine Carbonylgruppe als reaktive Gruppe, welche mit der CH-aciden Verbindung gemäß Formel I unter Ausbildung einer Kohlenstoff-Kohlenstoff-Bindung reagiert. Ferner sind erfindungsgemäß auch solche Verbindungen als Komponente B verwendbar, in denen die reaktive Carbonylgruppe derart derivatisiert ist, daß die Reaktivität des Kohlenstoffatoms der derivatisierten Carbonylgruppe gegenüber den CH-aciden Verbindungen der Formel I stets vorhanden ist. Diese Derivate sind bevorzugt Additionsverbindungen
a) von Aminen und deren Derivaten unter Bildung von Iminen oder Oximen als Additionsverbindung
b) von Alkoholen unter Bildung von Acetalen oder Ketalen als Additionsverbindung
c) wenn Komponente B sich von Aldehyden ableitet, von Wasser unter Bildung von Hydraten als Additionsverbindung
an das Kohlenstoffatom der Carbonylgruppe der reaktiven Carbonylverbindung.

Die Komponente B ist bevorzugt ausgewählt aus Verbindungen gemäß Formel III, 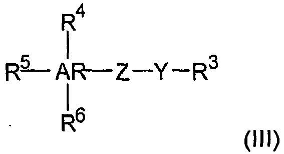 wobei
● AR steht für Benzol, Naphthalin, Pyridin, Pyrimidin, Pyrazin, Pyridazin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
● R³ steht für ein Wasserstoffatom, eine C₁-C₆-Alkyl-, C₂-C₆-Acyl-, C₂-C₆-Alkenyl-, C₁-C₄-Perfluoralkyl-, eine ggf. substituierte Aryl- oder Heteroarylgruppe,
● R⁴, R⁵ und R⁶ stehen unabhängig voneinander für ein Wasserstoffatom, ein Halogenatom, eine C₁-C₆-Alkyl-, C₁-C₆-Alkoxy-, C₁-C₆-Aminoalkyl-, C₁-C₆-Hydroxyalkylgruppe, eine C₁-C₆-Alkoxy-C₁-C₆-alkyloxygruppe, eine C₂-C₆-Acylgruppe, eine Acetyl-, Carboxyl-, Carboxylato-, Carbamoyl-, Sulfo-, Sulfato-, Sulfonamid-, Sulfonamido-, C₂-C₆-Alkenyl-, eine Aryl-, eine Aryl-C₁-C₆-alkylgruppe, eine Hydroxy-, eine Nitro-, eine Pyrrolidino-, eine Morpholino-, eine Piperidino-, eine Amino- bzw. Ammonio- oder eine 1-Imidazol(in)iogruppe, wobei die letzten drei Gruppen mit einer oder mehreren C₁-C₆-Alkyl-, C₁-C₆-Carboxyalkyl-, C₁-C₆-Hydroxyalkyl-, C₂-C₆-Alkenyl-, C₁-C₆-Alkoxy-C₁-C₆-alkyl-, mit ggf. substituierten Benzylgruppen, mit Sulfo-(C₁-C₄)-alkyl- oder Heterozyklus-(C₁-C₄)-alkylgruppen substituiert sein können,
   wobei auch zwei der Reste aus R⁴, R⁵, R⁶ und ―Z-Y-R³ zusammen mit dem Restmolekül einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R⁴, R⁵ und R⁶,
● Z steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei Z zusammen mit der -Y-R³-Gruppe auch einen gegebenenfalls substituierten 5-, 6- oder 7-Ring bilden kann,
● Y steht für eine Gruppe, die ausgewählt ist aus Carbonyl, einer Gruppe gemäß Formel IV und einer Gruppe gemäß Formel V 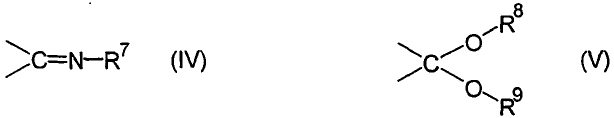 wobei
   ● R⁷ steht für ein Wasserstoffatom, eine Hydroxygruppe, eine C₁-C₄-Alkoxygruppe, eine C₁-C₆-Alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe,
   ● R⁸ und R⁹ stehen unabhängig voneinander für ein Wasserstoffatom, eine C₁-C₆-Alkylgruppe, eine Arylgruppe oder bilden gemeinsam zusammen mit dem Strukturelement O-C-O der Formel V einen 5- oder 6-gliederigen Ring.

Die Komponente B wird besonders bevorzugt ausgewählt aus der Gruppe bestehend aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4,5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxybenzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxybenzaldehyd, 3,4-Dihydroxy-5-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 3,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indoli-nyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchino-linium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- und/oder Aminosubstituenten, als Komponente B verwendet. Dabei werden wiederum die Verbindungen gemäß Formel VI bevorzugt, 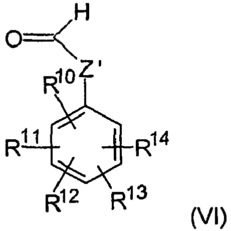 worin
● R¹⁰, R¹¹ und R¹² stehen unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe,
● Z' steht für eine direkte Bindung oder eine Vinylengruppe,
● R¹³ und R¹⁴ stehen für ein Wasserstoffatom oder bilden gemeinsam, zusammen mit dem Restmolekül einen 5- oder 6-gliederigen aromatischen oder aliphatischen Ring.

Ganz besonders bevorzugte Verbindungen der Komponente B werden ausgewählt aus der Gruppe bestehend aus Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxybenzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxybenzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,4-Dihydroxy-5-methoxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 3,4,5-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd und Piperonal.

In einer zweiten Ausführungsform kann es zur Erweiterung des Farbspektrums sowie zur Verbesserung der Echtheitseigenschaften vorteilhaft sein, den erfindungsgemäßen Mitteln neben mindestens einer Verbindung gemäß Formel I und mindestens einer reaktiven Carbonylverbindung (Komponente B) mindestens eine weitere Verbindung als Komponente C, ausgewählt aus (a) CH-aciden Verbindungen und (b) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, zuzusetzen.

Die CH-aciden Verbindungen der Komponente C sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3-Trimethylchinoxalinium-p-toluolsulfonat, 1,2-Dihydro-1,3,4,6-tetramethyl-2-oxo-pyrimidinium-chlorid, 1,2-Dihydro-1,3,4,6-tetramethyl-2-oxo-pyrimidinium-hydrogensulfat, 1,2-Dihydro-1,3,4-trimethyl-2-oxo-pyrimidinium-chlorid, 1,2-Dihydro-4,6-dimethyl-1,3-dipropyl-2-oxo-pyrimidinium-chlorid, 1,2-Dihydro-1,3,4,6-tetramethyl-2-thioxo-pyrimidinium-hydrogensulfat und 2-Dihydro-1,3,4,5,6-pentamethyl-2-oxo-pyrimidinium-chlorid.

Die primären und sekundären aromatischen Amine der Komponente C sind bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5-Diaminophenol, 2,5-Diaminoanisol, 2,5-Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxy-benzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlorbenzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel VII dargestellt sind 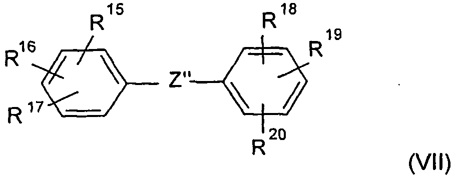 in der
● R¹⁵ für eine Hydroxy- oder eine Aminogruppe, die durch C₁-C₆-Alkyl-, C₁-C₆-Hydroxyalkyl-, C₁-C₆-Alkoxy- oder C₁-C₆-Alkoxy-C₁-C₆-alkylgruppen substituiert sein kann, steht,
● R¹⁶, R¹⁷, R¹⁸, R¹⁹ und R²⁰ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
● Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel VIII 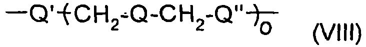 in der
   ● Q eine direkte Bindung, eine CH₂- oder CHOH-Gruppe bedeutet,
   ● Q' und Q" unabhängig voneinander für ein Sauerstoffatom, eine NR²¹-Gruppe, worin R²¹ ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
   ● o eine Zahl von 1 bis 4 bedeutet,
wie insbesondere 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die stickstoffhaltigen heterozyklischen Verbindungen der Komponente C sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-aminopyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterozyklische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Die aromatischen Hydroxyverbindungen der Komponente C sind bevorzugt ausgewählt aus der Gruppe bestehend aus 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylamino-phenol, 2-(2-Hydroxyethyl)phenol, 3,4-Methylendioxyphenol, 2,4-Dihydroxybenzoesäure, 3,4-Dihydroxybenzoesäure, 1-(2,4-Dihydroxyphenyl)essigsäure, 1-(3,4-Dihydroxy-phenyl)essigsäure, Gallussäure, 2,4,6-Trihydroxybenzoesäure, -acetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Aus der Druckschrift EP-A1-1 407 756 sind dem Fachmann Haarfärbemittel bekannt, die u.a. als direktziehenden Farbstoff mindestens einen Methinfarbstoff enthalten. In einer dritten Ausführungsform enthält das Färbemittel mindestens ein Reaktionsprodukt (im folgenden als Reaktionsprodukt RP bezeichnet) aus einer Verbindung der Formel I und einer Verbindung der Komponente B, insbesondere Verbindungen gemäß Formel III und Formel IV, als direktziehenden Farbstoff. Derartige Reaktionsprodukte RP können z. B. durch Erwärmen der beiden Reaktionspartner in wässrigem neutralen bis schwach alkalischen Milieu erhalten werden, wobei die Reaktionsprodukte RP entweder als Feststoff aus der Lösung ausfallen oder durch Eindampfen der Lösung daraus isoliert werden.

In den erfindungsgemäßen Mitteln können somit Reaktionsprodukte RP gemäß Formel IX enthalten sein, 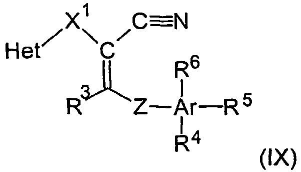 worin
● Het, X¹, Ar, R³, R⁴, R⁵, und R⁶ so definiert sind, wie unter Formel I beschrieben,
mit der Maßgabe, dass wenn R³ für ein Wasserstoffatom und Z für eine direkte Bindung und die Gruppierung ―[ArR⁴R⁵R⁶] der Formel IX für eine Gruppe 3,5-Dichlor-4-hydroxyphenyl stehen, Het nicht abgeleitet wird von Benzofuran, Thiazol oder Benzothiazol.

Zur Synthese der Reaktionsprodukte RP können Molverhältnisse der Komponente B zu der Verbindung gemäß Formel I von etwa 1:1 bis etwa 2:1 sinnvoll sein.

Es ist besonders bevorzugt, wenn die erfindungsgemäßen Mittel solche Reaktionsprodukte RP gemäß Formel IX enthalten, in denen AR gemäß Formel IX für Benzol oder Naphthalin steht.

Es ist besonders bevorzugt, dass R³ gemäß Formel IX ein Wasserstoffatom ist.

Ferner ist es besonders bevorzugt, wenn die erfindungsgemäßen Mittel solche Reaktionsprodukte RP gemäß Formel IX enthalten, in denen Z gemäß Formel IX für eine direkte Bindung oder Vinylen steht.

Als ganz besonders bevorzugte Reaktionsprodukte RP gemäß Formel IX ist gemäß dieser Ausführungsform in den erfindungsgemäßen Mitteln mindestens eine Verbindung ausgewählt aus der Gruppe bestehend aus 2-(2,5-Dimethyl-3-furoyl)-3-(4-hydroxy-3-methoxyphenyl)acrylonitril, 3-(2,4-Dihydroxyphenyl)-2-(2,5-dimethyl-3-furoyl)acrylonitril, 2-(2,5-Dimethyl-3-furoyl)-3-(4-hydroxy-3,5-dimethoxyphenyl)acrylonitril, 2-(2,5-Dimethyl-3-furoyl)-3-(4-hydroxyl-3,5-dimethylphenyl)acrylonitril, 2-(2,5-Dimethyl-3-furoyl)-3-(4-hydroxy-2-merthoxyphenyl)acrylonitril, 3-(3,4-Dihydroxyphenyl)-2-(2,5-dimethyl-3-furoyl)acrylonitril, 3-[4-(Diethylamino)phenyl]-2-(2,5-dimethyl-3-furoyl)acrylonitril, 2-(2,5-Dimethyl-3-furoyl)-3-(4-hydroxy-1-naphthyl)acrylonitril, 2-(2-Furoyl)-3-(1*H*-indol-3-yl)acrylonitril, 4-[2-Cyano-3-(2-furyl)-3-oxoprop-1-enyl]-1-methylchinolinium 4-Methylbenzolsulfonat, 4-[2-Cyano-3-(2-furyl)-3-oxoprop-1-enyl]-1-methylpyridinium Benzolsulfonat, 3-(2,4-Dihydroxyphenyl)-2-(2-furoyl)acrylonitril, 2-(2-Furoyl)-3-(4-hydroxy-3,5-dimethoxyphenyl)acrylonitril, 2-(2-Furoyl)-3-(4-hydroxy-3-methoxyphenyl)acrylonitril, 5-[4-(Diethylamino)phenyl]2-(2-furoyl)penta-2,4-diennitril, 2-(2-Furoyl)-5-(4-hydroxy-3-methoxyphenyl)penta-2,4-dienitril, 3-(2-Furyl)-3-oxo-2-(2-oxo-1,2-dihydro-3*H*-indol-3-ylidene)propannitril, 3-(2,4-Dihydroxyphenyl)-2-(thien-2-ylcarbonyl)acrylonitril, 3-(4-Hydroxy-3,5-dimethoxyphenyl)-2-(thien-2-ylcarbonyl)-acrylonitril, 3-(4-Hydroxy-3-methoxyphenyl)-2-(thien-2-ylcarbonyl)acrylonitril, 5-[4-(Diethylamino)phenyl]-2-(thien-2-ylcarbonyl)penta-2,4-diennitril, 5-(4-Hydroxy-3-methoxyphenyl)-2-(thien-2-ylcarbonyl)penta-2,4-diennitril, 3-oxo-2-(2-oxo-1,2-dihydro-3*H*-indol-3-ylidene)-3-thien-2-ylpropannitril, 3-(1*H*-indol-3-yl)-2-(thien-2-ylcarbonyl)acrylonitril, 4-[2-Cyano-3-oxo-3-thien-2-ylprop-1-enyl]-1-methylchinolinium 4-methylbenzolsulfonat, 4-[2-Cyano-3-oxo-3-thien-2-ylprop-1-enyl]-1-methylpyridinium benzolsulfonat, 2-(1,3-Benzolthiazol-2-yl)-3-(3,4-dihydroxyphenyl)acralonitril, 2-(1,3-Benzolthiazol-2-yl)-3-(2,4,5-trimethoxyphenyl)acrylonitril, 2-(1,3-Benzothiazol-2-yl)-3-[4-(diethylamino)phenyl]acrylonitril, 2-(1,3-Benzothiazol-2-yl)-3-[4-(diethylamino)-2-hydroxyphenyl]acrylonitril, 2-(1,3-Benzothiazol-2-yl)-3-(4-hydroxyphenyl)acrylonitril, 2-(1,3-Benzothiazol-2-yl)-3-(4-hydroxy-3,5-dimethylphenyl)acrylonitril, 2-(1,3-Benzothiazol-2-yl)-3-(4-hydroxy-2-methoxyphenyl)acrylonitril, 2-(1,3-Benzothiazol-2-yl)-3-(4-hydroxy-1-naphthly)acrylonitril, 1,3-Benzothiazol-2-yl(2-oxo-1,2-dihydro-3*H*-indol-3-ylidene(acetonitril, 2-(1,3-benzothiazol-2-yl)-3-(1*H*-indol-3-yl)acrylonitril, 2-(1,3-Benzothiazol-2-yl)-5-(4-hydroxy-3-methoxyphenyl)penta-2,4-dienenitril, 2-(1,3-Benzothiazol-2-yl)-3-(4-hydroxy-3,5-dimethoxyphenyl)acrylonitril, 2-(1,3-Benzothiazol-2-yl)-3-(2,4-dihydroxyphenyl)acrylonitril, 4-[2-(1,3-Benzothiazol-2-yl)-2-cyanovinyl]-1-methylpyridinium benzolsulfonat, 4-[2-(1,3-benzothiazol-2-yl)-2-cyanovinyl]-1-methylquinolinium methylbenzolsulfonat, (1,3-Benzothiazol-2-yl)-5-[4-(dimethylamino)phenyl]penta-2,4-diennitril, 2-(1,3-Benzothiazol-2-yl)-3-(4-hydroxy-3-methoxyphenyl)acrylonitril, 2-(1*H*-Benzimidazol-2-yl)-3-(3,4-dihydroxyphenyl)acrylonitril, 2-(1*H*-Benzimidazol-2-yl)-3-(2,4,5-trimethoxyphenyl)acrylonitril, 2-(1*H*-Benzimidazol-2-yl)-3-[4-diethylamino)phenyl]acrylonitril, 2-(1*H*-Benzimidazol-2-yl)-3-[4-(diethylamino)-2-hydroxyphenyl]acrylonitril, 2-(1*H*-Benzimidazol-2-yl)-3-(4-hydroxyphenyl)acrylonitril, 2-(1*H*-Benzimidazol-2-yl)-3-(4-hydroxy-3,5-diemethylphenyl)acrylonitril, 2-(1*H-*Benzimidazol-2-yl)-3-(4-hydroxy-2-methoxyphenyl)acrylonitril, 2-(1*H*-Benzimidazol-2-yl)-3-(4-hydroxy-1-naphthyl)acrylonitril, 1*H*-Benzimidazol-2-yl(2-oxo-1,2-dihydro-3*H*-indol-3-ylidene)acetonitril, 2-(1*H*-Benzimidazol-2-yl)-3-(1*H*-indol-3-yl)acrylonitril, 2-(1*H-*Benzimidazol-2-yl)-5-(4-hydroxy-3-methoxyphenyl)penta-2,4-diennitril, 2-(1*H-*Benzimidazol-2-yl)-3-(4-hydroxy-3,5-dimethoxyphenyl)acrylonitril, 2-(1*H*-Benzimidazol-2-yl)-3-(2,4-dihydroxyphenyl)acrylonitril, 4-[2-(1*H*-Benzimidazol-2-yl)-2-cyanovinyl]-1-methylpyridinium benzolsulfonat, 4-[2-(1*H*-Benzimidazol-2-yl)-2-cyanovinyl]-1-methylquinolinium 4-methylbenzolsulfonat, 2-(1*H*-Benzimidazol-2-yl)-5-[4-(dimethylamino)phenyl]penta-2,4-diennitril, 2-(1*H*-Benzimidazol-2-yl)-3-(4-hydroxy-3-methoxyphenyl)acrylonitril.

Die voranstehend genannten Verbindungen mit der Formel I, die Verbindungen der Komponente B, Komponente C sowie die Reaktionsprodukte RP werden jeweils vorzugsweise in einer Menge von 0,03 bis 65 mmol, insbesondere von 1 bis 40 mmol, bezogen auf 100 g des gesamten Färbemittels, verwendet.

In einer vierten Ausführungsform können die erfindungsgemäßen Mittel neben mindestens einer Verbindung gemäß Formel I und mindestens einer reaktiven Carbonylverbindung, mindestens eine Entwicklerkomponente und gegebenenfalls mindestens eine Kupplerkomponente als Oxidationsfarbstoffvorprodukte enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 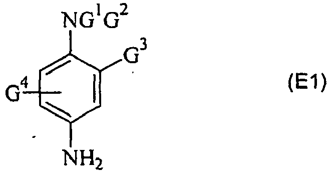 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁-bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-lsopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N-Ethyl-N-(β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 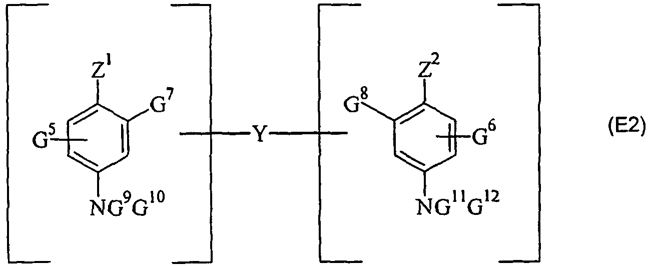 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 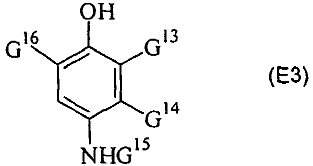 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(ß-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 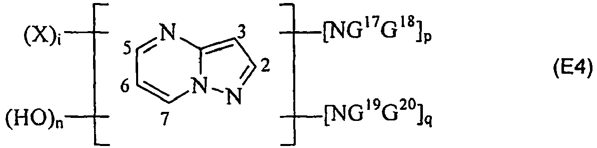 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

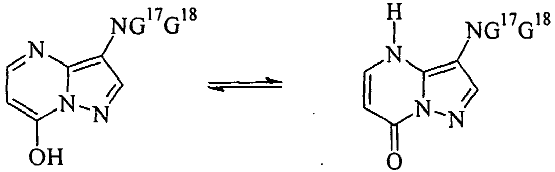

Unter den Pyrazolo[1,5-a]-pyrimidinen der obenstehenden Formel (E4) kann man insbesondere nennen:
- Pyrazolo[1,5-a]pyrimidin-3,7-diamin;
- 2,5-Dimethylpyrazolo[1,5-a]-pyrimidin-3,7-diamin;
- Pyrazolo[1,5-a]pyrimidin-3,5-diamin;
- 2,7-Dimethylpyrazolo[1,5-a]-pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

Die in den erfindungsgemäßen Mitteln optional enthaltenen Kupplerkomponenten sind bevorzugt ausgewählt aus
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe können im Rahmen einer fünften Ausführungsform ferner bevorzugt solche Indole und Indoline in den erfindungsgemäßen Mitteln eingesetzt werden, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel XIa, 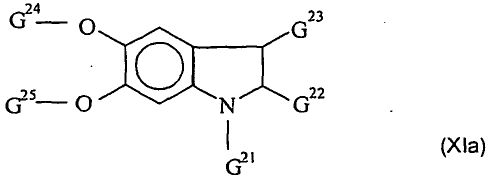 in der unabhängig voneinander
- G²¹ steht für Wasserstoff, eine C₁-C₄-Alkylgruppe oder eine C₁-C₄-Hydroxy-alkylgruppe,
- G²² steht für Wasserstoff oder eine -COOH-Gruppe, wobei die -COOH-Gruppe auch als Salz mit einem physiologisch verträglichen Kation vorliegen kann,
- G²³ steht für Wasserstoff oder eine C₁-C₄-Alkylgruppe,
- G²⁴ steht für Wasserstoff, eine C₁-C₄-Alkylgruppe oder eine Gruppe -CO-G²⁶, in der G²⁶ steht für eine C₁-C₄-Alkylgruppe, und
- G²⁵ steht für eine der unter G²⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel Xlb, 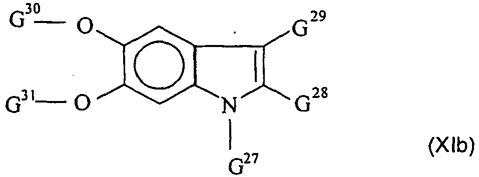 in der unabhängig voneinander
- G²⁷ steht für Wasserstoff, eine C₁-C₄-Alkylgruppe oder eine C₁-C₄-Hydroxyalkylgruppe,
- G²⁸ steht für Wasserstoff oder eine -COOH-Gruppe, wobei die -COOH-Gruppe auch als Salz mit einem physiologisch verträglichen Kation vorliegen kann,
- G²⁹ steht für Wasserstoff oder eine C₁-C₄-Alkylgruppe,
- G³⁰ steht für Wasserstoff, eine C₁-C₄-Alkylgruppe oder eine Gruppe -CO-G³², in der G³² steht für eine C₁-C₄-Alkylgruppe, und
- G³¹ steht für eine der unter G³⁰ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer echten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, wie Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel bevorzugt einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

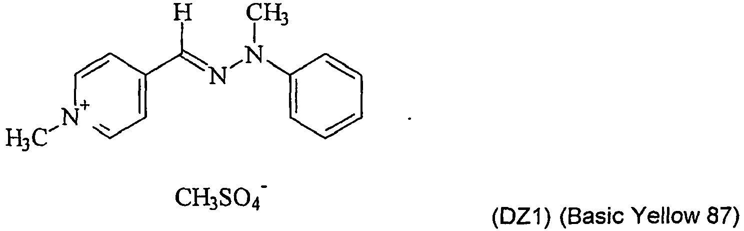

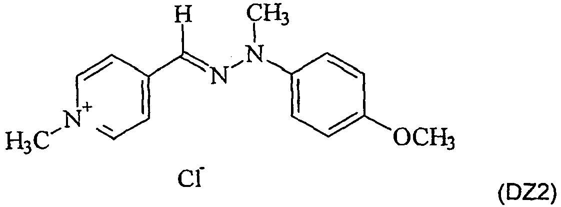

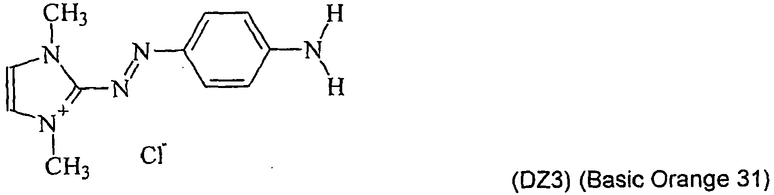

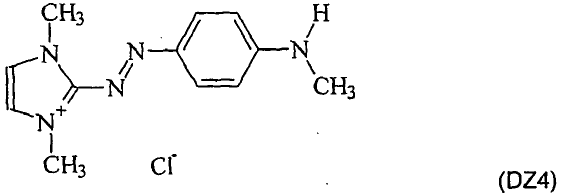

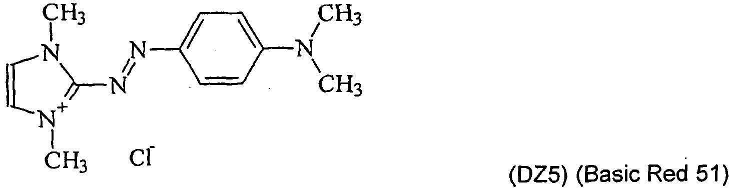

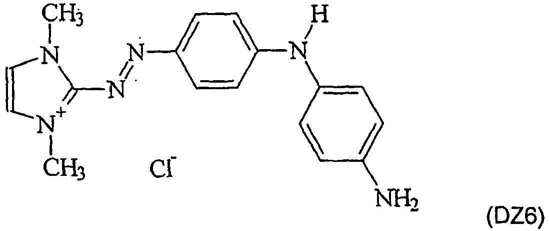

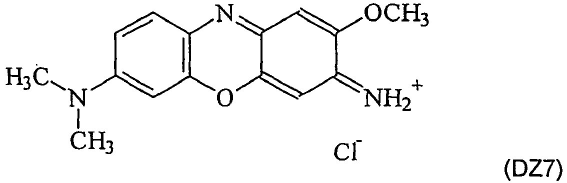

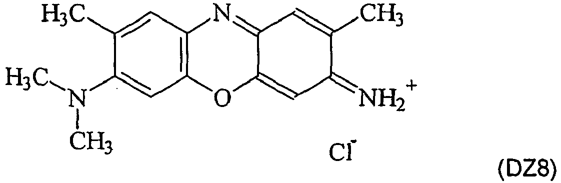

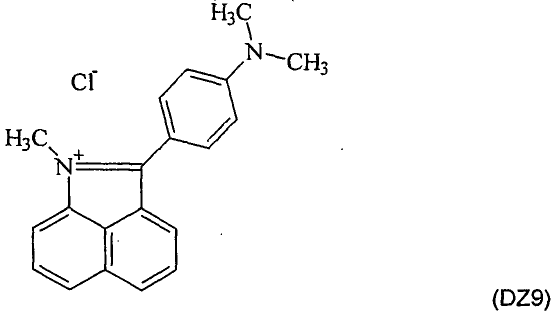

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, daß die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann, insbesondere wenn das erfindungsgemäße Mittel keine Oxidationsfarbstoffvorprodukte enthält, verzichtet werden. Wenn das erfindungsgemäße Mittel luftoxidable Oxidationsfarbstoffvorprodukte oder Indol bzw. Indolinderivate enthält, kann in einem solchen Fall ohne Probleme auf Oxidationsmittel verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus Iodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar werden die Färbemittel in einen wasserhaltigen kosmetischen Träger eingearbeitet. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise.das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein zweiter Gegenstand der vorliegenden Erfindung betrifft die Verwendung von mindestens einer Verbindung gemäß Formel I, 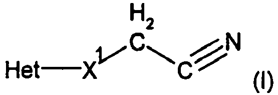 wobei Het und X¹ wie oben definiert sind, in Kombination mit mindestens einer reaktiven Carbonylverbindung als färbende Komponente in Haarfärbemitteln.

In einer bevorzugten Ausführungsform werden die Verbindungen gemäß Formel I in Kombination mit mindestens einer reaktiven Carbonylverbindung der Komponente B ausgewählt aus den vorstehend benannten bevorzugten und besonders bevorzugten Vertretern, als färbende Komponente in Haarfärbemitteln verwendet.

Darüber hinaus kann es bevorzugt sein, mindestens ein Reaktionsprodukt RP aus einer Verbindung gemäß Formel I und einer Verbindung der Komponente B als färbende Komponenten in Haarfärbemitteln zu verwenden.

Ein dritter Gegenstand der vorliegenden Erfindung betrifft ein Verfahren zum Färben von keratinhaltigen Fasern, insbesondere menschlichen Haaren, worin ein Färbemittel, enthaltend mindestens eine Verbindung gemäß Formel I, 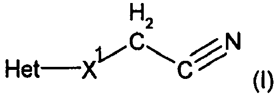 wobei Het und X¹ wie oben definiert sind in Kombination mit mindestens einer Verbindung mit einer reaktiven Carbonylgruppe (Komponente B), sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird. Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt.

Dabei können die Verbindungen gemäß Formel I und die Verbindungen der Komponente B, insbesondere deren vorstehend benannten bevorzugte und besonders bevorzugte Vertreter, als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Vor der Anwendung des erfindungsgemäßen Mittels in dem erfindungsgemäßen Verfahren kann es wünschenswert sein, die zu färbende keratinhaltige Faser einer Vorbehandlung zu unterziehen. Die zeitliche Abfolge des dazu erforderlichen Vorbehandlungsschritts und der Anwendung des erfindungsgemäßen Mittels muß nicht unmittelbar nacheinander sein, sondern es kann zwischen dem Vorbehandlungsschritt und der Anwendung des erfindungsgemäßen Mittels ein Zeitraum von bis maximal zwei Wochen liegen. Dazu eignen sich mehrere Vorbehandlungsmethoden. Bevorzugt wird die Faser
V1 vor der Anwendung des erfindungsgemäßen Mittels einer Blondierung oder
V2 vor der Anwendung des erfindungsgemäßen Mittels einer oxidativen Färbung
unterzogen.

Im Rahmen der Vorbehandlung V1 wird die keratinhaltige Faser mit einem Blondiermittel behandelt. Das Blondiermittel enthält neben einem Oxidationsmittel, wie üblicherweise Wasserstoffperoxid, bevorzugt mindestens ein als Oxidations- und Bleichverstärker wirksames anorganisches Persalz, wie z.B. ein Peroxodisulfat von Natrium, Kalium oder Ammonium. Färbungen gemäß des erfindungsgemäßen Verfahrens erhalten durch die Vorbehandlung V1 eine besondere Brillanz und Farbtiefe.

Im Rahmen der Vorbehandlung V2 wird ein Mittel enthaltend vorgenannte Oxidationsfarbstoffvorprodukte als Entwickler- und gegebenenfalls Kupplerkomponenten sowie gegebenenfalls vorgenannte Derivate des Indols bzw. Indolins auf die Faser aufgetragen und nach einer Einwirkzeit gegebenenfalls unter Zusatz von vorgenannten geeigneten Oxidationsmitteln auf dem Haar für 5-45 Minuten auf der Keratinfaser belassen. Danach wird das Haar gespült. Durch die anschließende Anwendung des erfindungsgemäßen Mittels kann vorhandenen Oxidationsfärbungen einen neue Farbnuance verliehen werden. Wählt man die Farbnuance des erfindungsgemäßen Mittels in der gleichen Farbnuance der oxidativen Färbung aus, so kann die Färbung vorhandener Oxidationsfärbungen nach dem erfindungsgemäßen Verfahren aufgefrischt werden. Es zeigt sich, daß die Farbauffrischung oder Nuancierung gemäß des erfindungsgemäßen Verfahrens einer Farbauffrischung bzw. Nuancierung allein mit herkömmlichen direktziehenden Farbstoffen in der Farbbrillanz und Farbtiefe überlegen ist.

Enthält das Haarfärbemittel neben den Verbindungen gemäß Formel I und der Komponente B zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die Verbindungen gemäß Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

Ein vierter Gegenstand der Erfindung ist die Verwendung von mindestens einer Verbindung gemäß Formel I, 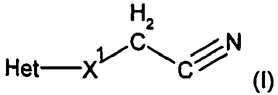 wobei Het und X¹ wie oben definiert sind, in Kombination mit reaktiven Carbonylverbindungen (Komponente B), zur Nuancierung von Oxidationsfärbungen von keratinhaltigen Fasern, insbesondere menschlichen Haaren. Bei der Verwendung ist es unerheblich, ob die Nuancierung gleichzeitig während der oxidativen Färbung erfolgt, oder die oxidative Färbung zeitlich vor der Nuancierung liegt.

Ein fünfter Gegenstand der Erfindung ist die Verwendung von mindestens einer Verbindung gemäß Formel I, 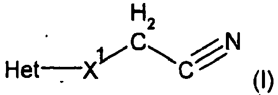 wobei Het und X¹ wie oben definiert sind in Kombination mit mindestens einer reaktiven Carbonylverbindung (Komponente B), zur Farbauffrischung von mit oxidativen Färbemitteln gefärbten keratinhaltigen Fasern.

Die Färbungen keratinhaltiger Fasern sind bekanntermaßen Umwelteinflüssen, wie beispielsweise Licht, Reibung oder Waschungen, ausgesetzt und können dadurch an Brillanz und Farbtiefe verlieren. Schlimmstenfalls stellt sich gegebenenfalls eine Nuancenverschiebung der Färbung ein. Solche gealterten Färbungen keratinhaltiger Fasern können, wenn der Anwender es wünscht, durch eine Farbauffrischung wieder annähernd in den farblichen Zustand versetzt werden, wie er sich unmittelbar nach der ursprünglichen Färbung präsentierte. Es ist erfindungsgemäß, für eine solche Farbauffrischung eine Kombination aus mindestens einer Verbindung mit der Formel I und mindestens einer reaktiven Carbonylverbindung zu verwenden.

### Beispiele

### 1.0 Herstellung einer Färbelösung

Es wurde je eine Aufschlämmung bzw. Lösung von 3 mmol der Verbindung gemäß Formel I (Komponente A) mit 0,41 g Natriumacetat in 30 ml Wasser bei ca. 50°C hergestellt. Unmittelbar vor der Anwendung werden zu dieser Mischung 3 mmol der Verbindungen der Komponente B hinzugefügt. Die Mischung wurde mit einer 10 %igen wäßrigen Natriumhydroxidlösung auf einen pH-Wert von pH 9 eingestellt (siehe Tabelle 1).

### 2.0 Ausfärbungen

In die frisch hergestellte Färbelösung wurde bei 30°C 30 Minuten lang eine Strähne zu 90% ergrauten, nicht vorbehandelten Menschenhaares (Firma Kerling, naturweiß) eingebracht. Die Strähne wurde dann 30 Sekunden mit lauwarmem Wasser gespült, mit warmer Luft (30°C bis 40°C) getrocknet und anschließend ausgekämmt.

Die jeweiligen Farbnuancen der Beispielausfärbungen sind in der nachfolgenden Tabelle 1 wiedergegeben.

### Verbindungen der Komponente A (zu Tabelle 1):

A1 2-(2-Thenoyl)-acetonitril
A2 2-(2-Furoyl)-acetonitril
A3 1*H*-Benzimidazol-2-ylacetonitril
A4 1*H*-Benzothiazol-2-ylacetonitril
A5 3-(2,5-Dimethyl-3-furyl)-3-oxopropannitril
A6 2-(2,5-Dimethylpyrrol-3-oyl)-acetonitril
A7 2-(1,2,5-Trimethylpyrrol-3-oyl)-acetonitril
A8 2-(2-Methyl-indol-3-oyl)-acetonitril
A9 2-(Indol-3-oyl)-acetonitril

### Verbindungen der Komponente B (zu Tabelle 1):

B1 2,4-Dihydroxybenzaldehyd
B2 4-Formyl-1-methyl-pyridinium-benzolsulfonat
B3 4-Formyl-1-methyl-chinolinium-p-toluolsulfonat
B4 4-Dimethylaminozimtaldehyd
B5 4-Hydroxy-3-methoxy-benzaldehyd
B6 Isatin
B7 Indol-3-carboxaldehyd
B8 Coniferylaldehyd
B9 3,5-Dimethoxy-4-hydroxybenzaldehyd
B10 3,5-Dimethyl-4-hydroxybenzaldehyd
B11 4-Hydroxy-2-methoxybenzaldehyd
B12 4-Hydroxy-1-naphthaldehyd
B13 3,4-Dihydroxybenzaldehyd
B14 2,4,5-Trimethoxybenzaldehyd
B15 4-Diethylaminobenzaldehyd
B16 4-Hydroxybenzaldehyd

**Tabelle 1**

| **Komponente A** | **Komponente B** | **Farbe** | **pH-Wert** |
|---|---|---|---|
| A1 | B1 | braungelb | 9 |
| A1 | B2 | beigebraun | 9 |
| A1 | B3 | beigebraun | 9 |
| A1 | B4 | dunkelrot | 9 |
| A1 | B5 | braunorange | 9 |
| A1 | B6 | grünbeige | 9 |
| A1 | B7 | hellgrün | 9 |
| A1 | B8 | schwarz | 9 |
| A1 | B9 | rot | 9 |
| A2 | B1 | braungelb | 9 |
| A2 | B2 | blassorange | 9 |
| A2 | B3 | braunorange | 9 |
| A2 | B4 | violettrot | 9 |
| A2 | B5 | leuchtend orange | 9 |
| A2 | B6 | blassorange | 9 |
| A2 | B7 | gelb | 9 |
| A2 | B8 | grau | 9 |
| A2 | B9 | leuchtend rot | 9 |
| A3 | B1 | neongelbgrün | 9 |
| A3 | B2 | braun | 9 |
| A3 | B3 | braun | 9 |
| A3 | B4 | orange | 9 |
| A3 | B5 | gelb | 9 |
| A3 | B6 | rot | 9 |
| A3 | B7 | blassgelb | 9 |
| A3 | B8 | orangerot | 9 |
| A3 | B9 | orangegelb | 9 |
| A3 | B10 | gelb | 9 |
| A3 | B11 | gelb | 9 |
| A3 | B12 | gelborange | 9 |
| A3 | B13 | braunorange | 9 |
| A3 | B14 | zitronengelb | 9 |
| A3 | B15 | braungelb | 9 |
| A4 | B1 | gelbgrün | 9 |
| A4 | B2 | braun | 9 |
| A4 | B3 | violett | 9 |
| A4 | B4 | rot | 9 |
| A4 | B5 | orange | 9 |
| A4 | B6 | orangebraun | 9 |
| A4 | B7 | gelb | 9 |
| A4 | B8 | rotbraun | 9 |
| A4 | B9 | leuchtend orangerot | 9 |
| A4 | B10 | orange | 9 |
| A4 | B11 | gelborange | 9 |
| A4 | B12 | rotbraun | 9 |
| A4 | B13 | rotbraun | 9 |
| A4 | B14 | gelb | 9 |
| A4 | B15 | braunorange | 9 |
| A4 | B16 | gelborange | 9 |
| A5 | B1 | gelb | 9 |
| A5 | B5 | gelborange | 9 |
| A5 | B9 | orange | 9 |
| A5 | B10 | gelborange | 9 |
| A5 | B11 | gelb | 9 |
| A5 | B12 | rotorange | 9 |
| A5 | B13 | rotorange | 9 |
| A5 | B15 | gelborange | 9 |
| A6 | B1 | beigegelb | 9 |
| A6 | B3 | violett | 9 |
| A6 | B5 | gelb | 9 |
| A6 | B9 | gelborange | 9 |
| A6 | B10 | gelb | 9 |
| A6 | B11 | gelb | 9 |
| A6 | B12 | beigeorange | 9 |
| A6 | B13 | orange | 9 |
| A7 | B1 | beigegelb | 9 |
| A7 | B3 | violett | 9 |
| A7 | B5 | gelb | 9 |
| A7 | B9 | gelborange | 9 |
| A7 | B10 | gelb | 9 |
| A7 | B11 | gelb | 9 |
| A7 | B12 | beigeorange | 9 |
| A7 | B13 | orange | 9 |
| A8 | B1 | hellgelb | 9 |
| A8 | B3 | violett | 9 |
| A8 | B5 | gelb | 9 |
| A8 | B9 | gelborange | 9 |
| A8 | B10 | gelb | 9 |
| A8 | B11 | hellgelb | 9 |
| A8 | B12 | gelbgrün | 9 |
| A8 | B13 | orangegelb | 9 |
| A9 | B5 | gelb | 9 |
| A9 | B9 | gelborange | 9 |
| A9 | B10 | blassgelb | 9 |
| A9 | B12 | beige | 9 |
| A9 | B13 | gelborange | 9 |

## Patentansprüche

1. Mittel zum Färben von keratinhaltigen Fasern, insbesondere menschlichen Haaren, enthaltend in einem wasserhaltigen, kosmetischen Träger als Komponente A mindestens eine Verbindung gemäß Formel I oder deren physiologisch verträgliches Salz, 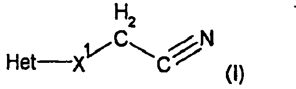 worin
• Het steht für einen gegebenenfalls substituierten Heteroaromaten,
• X¹ steht für eine direkte Bindung oder eine Carbonylgruppe,
und als Komponente B mindestens eine Verbindung mit einer reaktiven Carbonylgruppe.

2. Mittel nach Anspruch 1, **dadurch gekennzeichnet, daß** Rest Het steht für 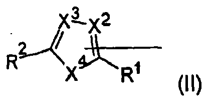 worin
• R¹ und R² stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylgruppe, eine Aryl-C₁-C₆-alkylgruppe, eine C₁-C₆-Hydroxyalkylgruppe, eine C₂₋C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{I}R^{II}N-(CH₂)ₘ-, worin R^{I} und R^{II} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{I} und R^{II} gemeinsam mit dem Stickstoffatom einen 5-, 6-oder 7-gliedrigen Ring bilden können und m steht für eine Zahl 0, 1, 2, 3 oder 4, wobei R¹ und/oder R² einen an den Ring des Restmoleküls ankondensierten, gegebenenfalls substituierten aromatischen oder heteroaromatischen, 5- oder 6-Ring bilden können
• X² und X³ stehen unabhängig voneinander für ein Stickstoffatom oder eine Gruppe CR³, wobei R³ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{III}R^{IV}N-(CH₂)ₙ-, worin R^{III} und R^{IV} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{III} und R^{IV} gemeinsam mit dem Stickstoffatom einen 5-, 6-oder 7-gliedrigen Ring bilden können und n steht für eine Zahl 0, 1, 2, 3 oder 4, wobei mindestens einer der Substituenten X² und X³ zusammen mit dem Restmolekül einen ankondensierten gegebenenfalls substituierten aromatischen 5- oder 6-Ring bilden kann,
• X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylgruppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{V}R^{VI}N-(CH₂)ₚ-steht, worin R^{V} und R^{VI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{V} und R^{VI} gemeinsam mit dem Stickstoffatom einen 5-, 6-oder 7-gliedrigen Ring bilden können und p steht für eine Zahl 0, 1, 2, 3 oder 4, substituiert sein können,
mit der Maßgabe, daß, wenn X⁴ für eine Vinylengruppe steht, mindestens eine der Gruppen X² oder X³ ein Stickstoffatom bedeutet.

3. Mittel nach einem der Ansprüche 1 oder 2, **dadurch gekennzeichnet, daß** sich der Rest Het ableitet von einem Heteroaromaten ausgewählt aus Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hadroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

4. Mittel nach einem der Ansprüche 1 bis 3, **dadurch gekennzeichnet, daß** die Verbindungen gemäß Formel I ausgewählt sind aus der Gruppe bestehend aus 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril.

5. Mittel nach einem der Ansprüche 1 bis 4, **dadurch gekennzeichnet, daß** die Komponente B ausgewählt ist aus Verbindung gemäß Formel III 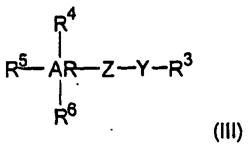 worin
• AR steht für Benzol, Naphthalin, Pyridin, Pyrimidin, Pyrazin, Pyridazin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quatemiert sein können,
• R³ steht für ein Wasserstoffatom, eine C₁-C₆-Alkyl-, C₂-C₆-Acyl-, C₂-C₆-Alkenyl-, C₁-C₄-Perfluoralkyl-, eine ggf. substituierte Aryl- oder Heteroarylgruppe,
• R⁴, R⁵ und R⁶ stehen unabhängig voneinander für ein Wasserstoffatom, ein Halogenatom, eine C₁-C₆-Alkyl-, C₁-C₆-Alkoxy-, C₁-C₆-Aminoalkyl-, C₁-C₆-Hydroxyalkylgruppe, eine C₁-C₆-Alkoxy-C₁-C₆-alkyloxygruppe, eine C₂-C₆-Acylgruppe, eine Acetyl-, Carboxyl-, Carboxylato-, Carbamoyl-, Sulfo-, Sulfato-, Sulfonamid-, Sulfonamido-, C₂-C₆-Alkenyl-, eine Aryl-, eine Aryl-C₁-C₆-alkylgruppe, eine Hydroxy-, eine Nitro-, eine Pyrrolidino-, eine Morpholino-, eine Piperidino-, eine Amino- bzw. Ammonio- oder eine 1-Imidazol(in)iogruppe, wobei die letzten drei Gruppen mit einer oder mehreren C₁-C₆-Alkyl-, C₁-C₆-Carboxyalkyl-, C₁-C₆-Hydroxyalkyl-, C₂-C₆-Alkenyl-, C₁-C₆-Alkoxy-C₁-C₆-alkyl-, mit ggf. substituierten Benzylgruppen, mit Sulfo-(C₁-C₄)-alkyl- oder Heterozyklus-(C₁-C₄)-alkylgruppen substituiert sein können,
wobei auch zwei der Reste aus R⁴, R⁵, R⁶ und -Z-Y-R³ zusammen mit dem Restmolekül einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R⁴, R⁵ und R⁶,
• Z steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei Z zusammen mit der -Y-R³-Gruppe auch einen gegebenenfalls substituierten 5-, 6- oder 7-Ring bilden kann,
• Y steht für eine Gruppe, die ausgewählt ist aus Carbonyl, einer Gruppe gemäß Formel IV und einer Gruppe gemäß Formel V, 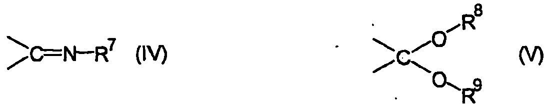 worin
• R⁷ steht für ein Wasserstoffatom, eine Hydroxygruppe, eine C₁-C₄-Alkoxygruppe, eine C₁-C₆-Alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe,
• R⁸ und R⁹ stehen unabhängig voneinander für ein Wasserstoffatom, eine C₁-C₆-Alkylgruppe, eine Arylgruppe oder bilden gemeinsam zusammen mit dem Strukturelement O-C-O der Formel V einen 5- oder 6-gliederigen Ring.

6. Mittel nach einem der Ansprüche 1 bis 5, **dadurch gekennzeichnet, daß** die Komponente B ausgewählt ist aus der Gruppe bestehend aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4,5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron,
Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,4-Dihydroxy-5-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 3,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal; 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, .2-(4'-Formylphenyl)-3-methylbenzothiazolium-, 2-(4'-Acetylphenyl)-3-methylbenzothiazolium-, 2-(4'-Formylphenyl)-3-methyl-benzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, - methansulfonat, -perchlorat, -sulfat, -chlorid, -bromid, -iodid, -tetrachlorozinkat, - methylsulfat-, trifluormethansulfonat, -tetrafluoroborat, Isatin, 1-Methyl-isatin, 1-Allylisatin, 1-Hydroxymethyl-isatin, 5-Chlor-isatin, 5-Methoxy-isatin, 5-Nitroisatin, 6-Nitroisatin, 5-Sulfo-isatin, 5-Carboxy-isatin, Chinisatin, 1-Methylchinisatin, sowie beliebigen Gemischen der voranstehenden Verbindungen.

7. Mittel nach einem der Ansprüche 1 bis 6, **dadurch gekennzeichnet, daß** die Komponente B ausgewählt ist aus der Gruppe bestehend aus Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxybenzaldehyd, 2-Ethoxy-4-hydroxy-benzatdehyd, 3-Ethoxy-4-hydroxy-benzaldehyd, 4-Ethoxy-2-hydroxy-benzaldehyd, 4-Ethoxy-3-hydr-oxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2.5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,4-Dihydroxy-5-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 3,4,5-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxyenzaldehyd, 4-(1-Imidazolyl)-benzaldehyd und Piperonal.

8. Mittel nach einem der Ansprüche 1 bis 7, **dadurch gekennzeichnet, daß** es mindestens eine Verbindung als Komponente C, ausgewählt aus (a) CH-aciden Verbindungen und (b) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, enthält.

9. Mittel nach Anspruch 9, **dadurch gekennzeichnet, daß** die CH-aciden Verbindungen der Komponente C ausgewählt sind aus der Gruppe bestehend aus 1,2,3,3-Tetramethyl-3H=indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3- Trimethylchinoxalinium-p-toluolsulfonat. 1,2-Dihydro-1,3,4,6-tetramethyl-2-oxo-pyrimidinium-chlorid, 1,2-Dihydro-1,3,4,6-tetramethyl-2-oxo-pyrimidinium-hydrogensulfat, 1,2-Dihydro-1,3,4-trimethyl-2-oxo-pyrimidinium-chlorid, 1,2-Dihydro-4,6-dimethyl-1,3-dipropyl-2-oxo-pyrimidinium-chlorid, 1,2-Dihydro-1,3,4,6-tetramethyl-2-thioxo-pyrimidinium-hydrogensulfat und 2-Dihydro-1,3,4,5,6-pentamethyl-2-oxo-pyrimidinium-chlorid.

10. Mittel nach Anspruch 8, **dadurch gekennzeichnet, daß** die primären und sekundären aromatischen Amine der Komponente C ausgewählt sind aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5-Diaminophenol, 2,5-Diaminoanisol, 2,5-Diaminophenethol, 4-Amino-3-methylphänol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäüre, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminopheny)-1,4-diazacycloheptan, aromatische Nitrile, wie 2-Amino-4-hydroxy-benzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amirio]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäüre, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest,
wie sie in der Formel VII dargestellt sind 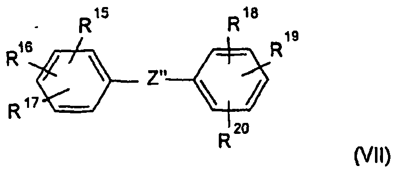 in der
● R¹⁵ für eine Hydroxy- öder eine Aminogruppe, die durch C₁-C₆-Alkyl-, C₁-C₆-Hydroxyalkyl-, C₁-C₆-Alkoxy- oder C₁-C₈-Alkoxy-C₁-C₆-alkylgruppen substituiert sein kann, steht,
● R¹⁶, R¹⁷, R¹⁸, R¹⁹ und R²⁰ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
● Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel VIII 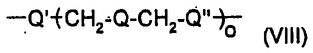 in der
● Q eine direkte Bindung, eine CH₂- oder CHOH-Gruppe bedeutet,
● Q' und Q" unabhängig voneinander für ein Sauerstoffatom, eine NR²¹-Gruppe, worin R²¹ ein Wasserstoffatom, eine C₂-C₆-Alkylgruppe oder C₁-C₆-Hydroxyalkylgruppe, wobei auch beide Gruppen zusammen mit dem Restmotekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
● o eine Zahl von 1 bis 4 bedeutet,
wie insbesondere 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-(N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

11. Mittel nach Anspruch 8, **dadurch gekennzeichnet, daß** die aromatischen Hydroxyverbindungen der Komponente C ausgewählt sind aus der Gruppe bestehend aus 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylamino-phenol, 2-(2-Hydroxyethyl)phenol, 3,4-Methylendioxyphenol, 2,4-Dihydroxybenzoesäure, 3,4-Dihydroxybenzoesäure, 1-(2,4-Dihydroxy-phenyl)essigsäure, 1-(3,4-Dihydroxy-phenyl)essigsäure, Gallussäure, 2,4,6-Trihydroxybenzoesäure, -acetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

12. Mittel nach Anspruch 8, **dadurch gekennzeichnet, daß** die stickstoffhaltigen heterozyklischen Verbindungen der Komponente C ausgewählt sind aus der Gruppe bestehend aus 2-Aminopyridin, 3-Aminopyridin, 4-Aminopyridin, 2-Amino-3-hydroxypyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und Hydroxypyrimidin-Derivate und die physiologisch verträglichen Salze der vorgenannten Verbindungen.

13. Mittel nach einem der Ansprüche 1 bis 12, **dadurch gekennzeichnet, daß** die Verbindungen der Formel I, die Verbindungen der Komponente B und die Verbindungen der Komponente C jeweils in einer Menge von 0,03 bis 65 mmol, insbesondere von 1 bis 40 mmol, bezogen auf 100 g des gesamten Färbemittels, enthalten sind.

14. Mittel nach einem der Ansprüche 1 bis 13, **dadurch gekennzeichnet, daß** es mindestens ein Reaktionsprodukt RP gemäß Formel IX enthält, 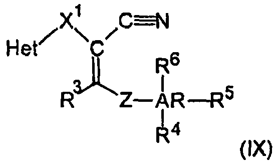 worin Het, X¹, R³, R⁴, R⁵, R⁶, AR und Z wie in Anspruch 5 definiert sind,
mit der Maßgabe, dass wenn R³ für ein Wasserstoffatom und Z für eine direkte Bindung und die Gruppierung -[ArR⁴R⁵R⁶] der Formel IX für eine Gruppe 3,5-Dichlor-4-hydroxyphenyl stehen, Het nicht abgeleitet wird von Benzofuran, Thiazol oder Benzothiazol.

15. Mittel nach Anspruch 14 **dadurch gekennzeichnet, daß** AR gemäß Formel IX für Benzol oder Naphthalin steht.

16. Mittel nach einem der Ansprüche 14 bis 15, **dadurch gekennzeichnet, daß** Z gemäß Formel IX für eine direkte Bindung oder Vinylen steht.

17. Mittel nach einem der Ansprüche 14 bis 16, **dadurch gekennzeichnet, daß** R³ gemäß Formel IX für ein Wasserstoffatom steht.

18. Mittel nach einem der Ansprüche 14 bis 17, **dadurch gekennzeichnet, daß** das Reaktionsprodukt RP in einer Menge von 0,03 bis 65 mmol, insbesondere von 1 bis 40 mmol, bezogen auf 100 g des gesamten Färbemittels, enthalten ist.

19. Mittel nach einem der Ansprüche 1 bis 18, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

20. Mittel nach einem der Ansprüche 1 bis 19, **dadurch gekennzeichnet, daß** es mindestens einen direktziehenden Farbstoff, vorzugsweise in einer Menge von 0,01 bis 20 Gew.-%, bezogen auf das gesamte Färbemittel, enthält.

21. Mittel nach einem der Ansprüche 1 bis 20, **dadurch gekennzeichnet, daß** es mindestens eine Entwicklerkomponente und gegebenenfalls mindestens eine Kupplerkomponente als Oxidationsfarbstoffvorprodukt enthält.

22. Mittel nach einem der Ansprüche 1 bis 21, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

23. Mittel nach einem der Ansprüche 1 bis 22, **dadurch gekennzeichnet, daß** es Oxidationsmittel, insbesondere H₂O₂, in einer Menge von 0,01 bis 6 Gew.-%, bezogen auf die Anwendungslösung, enthält.

24. Mittel nach einem der Ansprüche 1 bis 23, **dadurch gekennzeichnet, daß** es anionische, zwitterionische oder nichtionische Tenside enthält.

25. Verwendung von mindestens einer Verbindung gemäß Formel I gemäß Anspruch 1, in Kombination mit mindestens einer reaktiven Carbonylverbindung, als eine färbende Komponente in Haarfärbemitteln.

26. Verfahren zum Färben von keratinhaltigen Fasern, insbesondere menschlichen Haaren, worin ein Färbemittel, gemäß Anspruch 1, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

27. Verfahren gemäß Anspruch 26, **dadurch gekennzeichnet, daß** in einem Zweischrittverfahren die Komponente B vor oder nach Applikation der Verbindung gemäß Formel I auf die keratinhaltigen Fasern aufgebracht, die auf dem Haar erhaltene Mischung einige Zeit, üblicherweise ca. 15-30 Minuten, auf der Faser belassen und anschließend wieder ausgespült oder mit einem Shampoo ausgewaschen wird.

28. Verfahren nach einem der Ansprüche 26 oder 27, **dadurch gekennzeichnet, daß** die keratinhaltigen Fasern, bevor ein Färbemittel gemäß Anspruch 1 zur Anwendung kommt, im Rahmen einer Vorbehandlung mit einem Blondiermittel blondiert oder mit einem Oxidationsfärbemittel gefärbt wurden.

29. Verwendung von mindestens einer Verbindung gemäß Formel I, 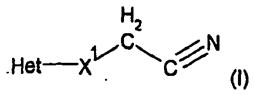 wobei Het und X¹ wie in Anspruch 1 definiert sind, in Kombination mit reaktiven Carbonylverbindungen (Komponente B), zur Nuancierung von Oxidationsfärbungen von keratinhaltigen Fasern, insbesondere menschlichen Haaren.

30. Verwendung von mindestens einer Verbindung gemäß Formel I, 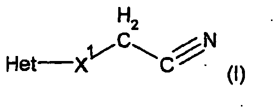 wobei Het und X¹ wie in Anspruch 1 definiert sind, in Kombination mit reaktiven Carbonylverbindungen (Komponente B), zur Farbauffrischung von mit oxidativen Färbemitteln gefärbten keratinhaltigen Fasern.

## Claims

1. An agent for dyeing keratin-containing fibres, in particular human hair, containing in a hydrous, cosmetic carrier as component A at least one compound according to formula I or the physiologically acceptable salt thereof, 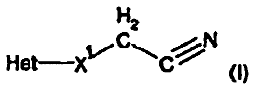 in which
● Het denotes an optionally substituted heteroaromatic,
● X¹ denotes a direct bond or a carbonyl group,
and as component B at least compound with a reactive carbonyl group.

2. An agent according to claim 1, **characterised in that** the residue Het denotes 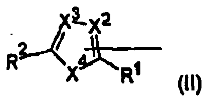 in which
● R¹ and R² mutually independently denote a hydrogen atom, a hydroxyl group, a halogen atom, a nitro group, a linear or cyclic C₁-C₆ alkyl group, a C₂-C₆ alkenyl group, an optionally substituted aryl group, a cyanomethyl group, a cyanomethylcarbonyl group, an optionally substituted heteroaryl group, an aryl-C₁-C₆-alkyl group, a C₁-C₆ hydroxyalkyl group, a C₂-C₆ polyhydroxyalkyl group, a C₁-C₆ alkoxy group, a C₁-C₆ alkoxycarbonyl group, a C₁-C₆-alkoxy-C₂-C₆-alkyl group, a C₁-C₆ sulfoalkyl group, a C₁-C₆ carboxyalkyl group, an R^{I}R^{II}N-(CH₂)ₘ group, in which R^{I} and R^{II} mutually independently denote a hydrogen atom, a linear or cyclic C₁-C₆ alkyl group, a C₂-C₆ alkenyl group, a C₁-C₆ hydroxyalkyl group or an aryl-C₁-C₄-alkyl group, wherein R^{I} and R^{II}, together with the nitrogen atom, may form a 5-, 6- or 7-membered ring and M denotes a number 0, 1, 2, 3 or 4, wherein R¹ and/or R² may form an optionally substituted aromatic or heteroaromatic, 5- or 6-membered ring fused to the ring of the remainder of the molecule
● X² and X³ mutually independently denote a nitrogen atom or a CR³ group, wherein R³ denotes a hydrogen atom, a hydroxyl group, a halogen atom, a nitro group, a linear or cyclic C₁-C₆ alkyl group, a C₂-C₆ alkenyl group, an optionally substituted aryl group, a cyanomethyl group, a cyanomethylcarbonyl group, an optionally substituted heteroaryl group, an aryl-C₁-C₆-alkyl group, a C₁-C₆ hydroxyalkyl group, a C₂-C₆ polyhydroxyalkyl group, a C₁-C₆ alkoxy group, a C₁-C₆ alkoxycarbonyl group, a C₁-C₆-alkoxy-C₂-C₆-alkyl group, a C₁-C₆ sulfoalkyl group, a C₁-C₆ carboxyalkyl group, and an R^{III}R^{IV}N-(CH₂)ₙ group, in which R^{III} and R^{IV} mutually independently denote a hydrogen atom, a linear or cyclic C₁-C₆ alkyl group, a C₂-C₆ alkenyl group, a C₁-C₆ hydroxyalkyl group or an aryl-C₁-C₄-alkyl group, wherein R^{III} and R^{IV}, together with the nitrogen atom, may form a 5-, 6- or 7-membered nitrogen atom and n denotes a number 0, 1, 2, 3 or 4, wherein at least one of the substituents X² and X³, together with the remainder of the molecule, may form a fused, optionally substituted aromatic 5- or 6-membered ring,
● X⁴ denotes an oxygen atom, a sulfur atom, a vinylene group or an N-H group, wherein the latter two stated groups may mutually independently be substituted with a linear or cyclic C₁-C₆ alkyl group, a C₂-C₆ alkenyl group, an optionally substituted aryl group, an optionally substituted heteroaryl group, an aryl-C₁-C₆-alkyl group, a C₂-C₆ hydroxyalkyl group, a C₂-C₆ polyhydroxyalkyl group, a C₁-C₆-alkoxy-C₂-C₆-alkyl group, a C₁-C₆ sulfoalkyl group, a C₁-C₆ carboxyalkyl group, an R^{V}R^{VI}N-(CH₂)ₚ group, in which R^{V} and R^{VI} mutually independently denote a hydrogen atom, a linear or cyclic C₁-C₆ alkyl group, a C₂-C₆ alkenyl group, a C₁-C₆ hydroxyalkyl group or an aryl-C₁-C₄-alkyl group, wherein R^{V} and R^{VI}, together with the nitrogen atom, may form a 5-, 6- or 7-membered ring and p denotes a number 0, 1, 2, 3 or 4,
with the proviso that, if X⁴ denotes a vinylene group, at least one of the X² or X³ groups means a nitrogen atom.

3. An agent according to either one of claims 1 or 2, **characterised in that** the residue Het is derived from a heteroaromatic selected from furan, thiophene, pyrrole, isoxazole, isothiazole, imidazole, oxazole, thiazole, pyridine, pyridazine, pyrimidine, pyrazine, 1,2,3-triazine, 1,2,4-triazine, 1,3,5-triazine, benzopyrrole, benzofuran, benzothiophene, benzimidazole, benzoxazole, indazole, benzoisoxazole, benzoisothiazole, indole, quinoline, isoquinoline, cinnoline, phthalazine, quinazoline, quinoxaline, acridine, benzoquinoline, benzoisoquinoline, phenazine, benzocinnoline, benzoquinazoline, benzoquinoxaline, phenoxazine, phenothiazine, nephthyridine, phenanthroline, indolizine, quinolizine, carboline, purine, pteridine and coumarin, wherein the above-stated heteroaromatics may be substituted with at least one group selected from a halogen atom, a nitro group, a thio group, a thio-(C₁-C₆)-alkyl group, a heteroaryl group, an aryl group, a (C₁-C₆) alkyl group, a (C₁-C₆) alkoxy group, a hydroxyl group, a (C₂-C₈) hydroxyalkyl group, a (C₂-C₆) polyhydroxyalkyl group, a (C₁-C₆)-alkoxyl-(C₁-C₆)-alkyl group, an aryl-(C₁-C₆)-alkyl group, an amino group, a (C₁-C₆) monoalkylamino group, a (C₁-C₆) dialkylamino group, a -(CH₂)ₙ-NR'R" dialkylaminoalkyl group, in which n is an integer of 2 and 6 and R' and R" mutually independently mean a linear or branched alkyl group, which may optionally together form a ring.

4. An agent according to any one of claims 1 to 3, **characterised in that** the compounds according to formula I are selected from the group consisting of 2-(5-bromo-2-furoyl)-acetonitrile, 2-(5-methyl-2-trifluoromethyl-3-furoyl)-acetonitrile, 3-(2,5-dimethyl-3-furyl)-3-oxopropanenitrile, 2-(2-thenoyl)-acetonitrile, 2-(3-thenoyl)-acetonitrile, 2-(5-fluoro-2-thenoyl)-acetonitrile, 2-(5-chloro-2-thenoyl)-acetonitrile, 2-(5-bromo-2-thenoyl)-acetonitrile, 2-(5-methyl-2-thenoyl)-acetonitrile, 2-(2,5-dimethylpyrrol-3-oyl)-acetonitrile, 2-(1,2,5-trimethylpyrrol-3-oyl)-acetonitrile, 1 H-benzimidazol-2-ylacetonitrile, 1*H*-benzothiazol-2-ylacetonitrile, 2-(pyrid-2-yl)-acetonitrile, 2,6-bis(cyanomethyl)-pyridine, 2-(indol-3-oyl)-acetonitrile, 2-(2-methylindol-3-oyl)-acetonitrile, 8-cyanoacetyl-7-methoxy-4-methylcoumarin, 2-(2-isopropyl-5,6-benzoquinolin-4-oyl)-acetonitrile, 2-(2-phenyl-5,6-benzoquinolin-4-oyl)-acetonitrile, 2-(quinoxalin-2-yl)-acetonitrile, 2-(coumaron-2-yl)-acetonitrile, 6,7-dichloro-5-(cyanoacetyl)-2,3-dihydro-1-benzofuran-2-carboxylic acid tert.-butyl ester, 2-(6-hydroxy-4,7-dimethoxy-1-benzofuran-5-oyl)-acetonitrile and 2-(1-phenyl-1,4-dihydrothiochromeno[4,3c]pyrazol-3-oyl)-acetonitrile.

5. An agent according to any one of claims 1 to 4, **characterised in that** component B is selected from compounds according to formula III 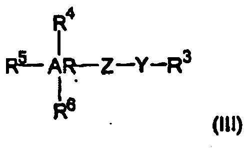 in which
● AR denotes benzene, naphthalene, pyridine, pyrimidine, pyrazine, pyridazine, carbazole, pyrrole, pyrazole, furan, thiophene, 1,2,3-triazine, 1,3,5-triazine, quinoline, isoquinoline, indole, indoline, indolizine, indan, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, wherein the N-heteroaromatics may also be quaternised,
● R³ denotes a hydrogen atom, a C₁-C₆ alkyl, C₂-C₆ acyl, C₂-C₆ alkenyl, C₁-C₄ perfluoroalkyl, an optionally substituted aryl or heteroaryl group,
● R⁴, R⁵ and R⁶ mutually independently denote a hydrogen atom, a halogen atom, a C₁-C₆ alkyl, C₁-C₆ alkoxy, C₁-C₆ aminoalkyl, C₁-C₆ hydroxyalkyl group, a C₁-C₆-alkoxy-C₁-C₆-alkyloxy group, a C₂-C₆ acyl group, an acetyl, carboxyl, carboxylato, carbamoyl, sulfo, sulfato, sulfonamide, sulfonamido, C₂-C₆ alkenyl, an aryl, an aryl-C₁-C₆-alkyl group, a hydroxyl, a nitro, a pyrrolidino, a morpholino, a piperidino, an amino or ammonio or a 1-imidazol(in)io group, wherein the latter three groups may be substituted with one or more C₁-C₆ alkyl, C₁-C₈ carboxyalkyl, C₁-C₆ hydroxyalkyl, C₂-C₆ alkenyl, C₁-C₆-alkoxy-C₁-C₆-alkyl, with optionally substituted benzyl groups, with sulfo-(C₁-C₄)-alkyl or heterocycle-(C₁-C₄)-alkyl groups,
wherein two residues from R⁴, R⁵, R⁶ and -Z-Y-R³, together with the remainder of the molecule, may also form a fused optionally substituted 5-, 6- or 7-membered ring, which may likewise bear a fused aromatic ring, wherein, as a function of the size of the ring, the AR system may bear further substituents which may mutually independently denote the same groups as R⁴, R⁵ and R⁶,
● Z denotes a direct bond, a carbonyl, a carboxy-(C₁-C₄)-alkylene, an optionally substituted C₂-C₆ alkenylene, C₄-C₆ alkadienylene, furylene, thienylene, arylene, vinylenearylene, vinylenefurylene, vinylenethienylene group, wherein Z, together with the -Y-R³ group, may also form an optionally substituted 5-, 6- or 7-membered ring,
● Y denotes a group which is selected from carbonyl, a group according to formula IV and a group according to formula V, 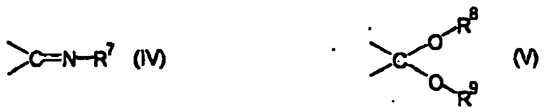 in which
● R⁷ denotes a hydrogen atom, a hydroxyl group, a C₁-C₄ alkoxy group, a C₁-C₆ alkyl group, a C₁-C₆ hydroxyalkyl group, a C₂-C₆ polyhydroxyalkyl group, a C₁-C₆-alkoxy-C₁-C₆-alkyl group,
● R⁸ and R⁹ mutually independently denote a hydrogen atom, a C₁-C₆ alkyl group, an aryl group, or, together with the structural element O-C-O of formula V, jointly form a 5- or 6-membered ring.

6. An agent according to any one of claims 1 to 5, **characterised in that** component B is selected from the group consisting of acetophenone, propiophenone, 2-hydroxyacetophenone, 3-hydroxyacetophenone, 4-hydroxyacetophenone, 2-hydroxypropiophenone, 3-hydroxypropiophenone, 4-hydroxypropiophenone, 2-hydroxybutyrophenone, 3-hydroxybutyrophenone, 4-hydroxybutyrophenone, 2,4-dihydroxyacetophenone, 2,5-dihydroxyacetophenone, 2,6-dihydroxyacetophenone, 2,3,4-trihydroxyacetophenone, 3,4,5-trihydroxyacetophenone, 2,4,6-trihydroxyacetophenone, 2,4,6-trimethoxyacetophenone, 3,4,5-trimethoxyacetophenone, 3,4,5-trimethoxyacetophenone diethyl ketal, 4-hydroxy-3-methoxyacetophenone, 3,5-dimethoxy-4-hydroxyacetophenone, 4-aminoacetophenone, 4-dimethylaminoacetophenone, 4-morpholinoacetophenone, 4-piperidinoacetophenone, 4-imidazolinoacetophenone, 2-hydroxy-5-bromoacetophenone, 4-hydroxy-3-nitroacetophenone, acetophenone-2-carboxylic acid, acetophenone-4-carboxylic acid, benzophenone, 4-hydroxybenzophenone, 2-aminobenzophenone, 4,4'-dihydroxybenzophenone, 2,4-dihydroxybenzophenone, 2,4,4-trihydroxybenzophenone, 2,3,4-trihydroxybenzophenone, 2-hydroxy-1-acetonaphthone, 1-hydroxy-2-acetonaphthone, chromone, chromone-2-carboxylic acid, flavone, 3-hydroxyflavone, 3,5,7-trihydroxyflavone, 4,5,7-trihydroxyflavone, 5,6,7-trihydroxyflavone, quercetin, 1-indanone, 9-fluorenone, 3-hydroxyfluorenone, anthrone, 1,8-dihydroxyanthrone, vanillin, coniferyl aldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-3-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 4-hydroxy-3,5-dimethoxybenzaldehyde, 4-hydroxy-3,5-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaldehyde, 3,4-dihydroxy-6-methylbenzaldehyde, 3,4-dihydroxy-2-methoxybenzaldehyde, 3,4-dihydroxy-5-methoxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 3,4,5-trihydroxybenzaldehyde, 2,5,6-trihydroxybenzaldehyde, 4-hydroxy-2-methoxybenzaldehyde, 4-dimethylaminobenzaldehyde, 4-diethylaminobenzaldehyde, 4-dimethylamino-2-hydroxybenzaldehyde, 4-diethylamino-2-hydroxybenzaldehyde, 4-pyrrolidinobenzaldehyde, 4-morpholinobenzaldehyde, 2-morpholinobenzaldehyde, 4-piperidinobenzaldehyde, 2-methoxy-1-naphthaldehyde, 4-methoxy-1-naphthaldehyde, 2-hydroxy-1-naphthaldehyde, 2,4-dihydroxy-1-napthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-hydroxy-1-naphthaldehyde, 4-dimethylamino-1-naphthaldehyde, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 4-dimethylamino-2-methoxybenzaldehyde, 4-(1-imidazolyl)-benzaldehyde, piperonal, 2,3,6,7-tetrahydro-1*H*,5*H*-benzo[ij]quinolizine-9-carboxaldehyde, 2,3,6,7-tetrahydro-8-hydroxy-1*H*,5*H*-benzo [ij]quinolizine-9-carboxaldehyde, N-ethylcarbazole-3-aldehyde, 2-formylmethylene-1,3,3-trimethylindoline (Fischer's aldehyde or tribasic aldehyde), 2-indolealdehyde, 3-indolealdehyde, 1-methylindole-3-aldehyde, 2-methylindole-3-aldehyde, 1-acetylindole-3-aldehyde, 3-acetylindole, 1-methyl-3-acetylindole, 2-(1',3',3'-trimethyl-2-indolinylidene)-acetaldehyde, 1-methylpyrrole-2-aldehyde, 1-methyl-2-acetylpyrrole, 4-pyridinealdehyde, 2-pyridinealdehyde, 3-pyridinealdehyde, 4-acetylpyridine, 2-acetylpyridine, 3-acetylpyridine, pyridoxal, quinoline-3-aldehyde, quinoline-4-aldehyde, antipyrine-4-aldehyde, furfural, 5-nitrofurfural, 2-thenoyltrifluoroacetone, chromone-3-aldehyde, 3-(5'-nitro-2'-furyl)-acrolein, 3-(2'-furyl)-acrolein and imidazole-2-aldehyde, 1,3-diacetylbenzene, 1,4-diacetylbenzene, 1,3,5-triacetylbenzene, 2-benzoylacetophenone, 2-(4'-methoxybenzoyl)-acetophenone, 2-(2'-furoyl)-acetophenone, 2-(2'-pyridoyl)-acetophenone and 2-(3'-pyridoyl)-acetophenone, benzylideneacetone, 4-hydroxybenzylideneacetone, 2-hydroxybenzylideneacetone, 4-methoxybenzylideneacetone, 4-hydroxy-3-methoxybenzylideneacetone, 4-dimethylaminobenzylideneacetone, 3,4-methylenedioxybenzylideneacetone, 4-pyrrolidinobenzylideneacetone, 4-piperidinobenzylideneacetone, 4-morpholinobenzylideneacetone, 4-diethylaminobenzylideneacetone, 3-benzylidene-2,4-pentanedione, 3-(4'-hydroxybenzylidene)-2,4-pentanedione, 3-(4'-dimethylaminobenzylidene)-2,4-pentanedione, 2-benzylidenecyclohexanone, 2-(4'-hydroxybenzylidene)-cyclohexanone, 2-(4'-dimethylaminobenzylidene)-cyclohexanone, 2-benzylidene-1,3-cyclohexanedione, 2-(4'-hydroxybenzylidene)-1,3-cyclohexanedione, 3-(4'-dimethylaminobenzylidene)-1,3-cyclohexanedione, 2-benzylidene-5,5-dimethyl-1,3-cyclohexanedione, 2-(4'-hydroxybenzylidene)-5,5-dimethyl-1,3-cyclohexanedione, 2-(4'-hydroxy-3-methoxybenzylidene)-5,5-dimethyl-1,3-cyclohexanedione, 2-(4'-dimethylaminobenzylidene)-5,5-dimethyl-1,3-cyclohexanedione, 2-benzylidencyclopentanone, 2'-(4-hydroxybenzylidene)-cyclopentanone, 2-(4'-dimethylaminobenzylidene)-cyclopentanone, 5-(4-dimethylaminophenyl)penta-2,4-dienal, 5-(4-diethylaminophenyl)penta-2,4-dienal, 5-(4-methoxyphenyl)penta-2,4-dienal, 5-(3,4-dimethoxyphenyl)penta-2,4-dienal, 5-(2,4-dimethoxyphenyl)penta-2,4-dienal, 5-(4-piperidinophenyl)penta-2,4-dienal, 5-(4-morpholinophenyl)penta-2,4-dienal, 5-(4-pyrrolidinophenyl)penta-2,4-dienal, 6-(4-dimethylaminophenyl)hexa-3,5-dien-2-one, 6-(4-diethylaminophenyl)hexa-3,5-dien-2-one, 6-(4-methoxyphenyl)hexa-3,5-dien-2-one, 6-(3,4-dimethoxyphenyl)hexa-3,5-dien-2-one, 6-(2,4-dimethoxyphenyl)hexa-3,5-dien-2-one, 6-(4-piperidinophenyl)hexa-3,5-dien-2-one, 6-(4-morpholinophenyl)hexa-3,5-dien-2-one, 6-(4-pyrrolidinophenyl)hexa-3,5-dien-2-one, 5-(4-dimethylamino-1-naphthyl)penta-3,5-dienal, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 4-hydroxy-3-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 6-nitropiperonal, 2-nitropiperonal, 5-nitrovanillin, 2,5-dinitrosalicylaldehyde, 5-bromo-3-nitrosalicylaldehyde, 3-nitro-4-formyl-benzenesulfonic acid, 4-nitro-1-naphthaldehyde, 2-nitrocinnamaldehyde, 3-nitrocinnamaldehyde, 4-nitrocinnamaldehyde, 9-methyl-3-carbazole-aldehyde, 9-ethyl-3-carbazolealdehyde, 3-acetylcarbazole, 3,6-diacetyl-9-ethylcarbazole, 3-acetyl-9-methylcarbazole, 1,4-dimethyl-3-carbazole-aldehyde, 1,4,9-trimethyl-3-carbazolealdehyde, 4-formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 4-acetyl-1-methylpyridinium, 2-acetyl-1-methylpyridinium, 4-acetyl-1-methylquinolinium, 5-formyl-1-methylquinolinium, 6-formyl-1-methylquinolinium, 7-formyl-1-methylquinolinium, 8-formyl-1-methylquinolinium, 5-formyl-1-ethylquinolinium, 6-formyl-1-ethylquinolinium, 7-formyl-1-ethylquinolinium, 8-formyl-1-ethylquinolinium, 5-formyl-1-benzylquinolinium, 6-formyl-1-benzylquinolinium, 7-formyl-1-benzylquinolinium, 8-formyl-1-benzylquinolinium, 5-formyl-1-allylquinolinium, 6-formyl-1-allylquinolinium, 7-formyl-1-allylquinolinium and 8-formyl-1-allylquinolinium, 5-acetyl-1-methylquinolinium, 6-acetyl-1-methylquinolinium, 7-acetyl-1-methylquinolinium, 8-acetyl-1-methylquinolinium, 5-acetyl-1-ethylquinolinium, 6-acetyl-1-ethylquinolinium, 7-acetyl-1-ethylquinolinium, 8-acetyl-1-ethylquinolinium, 5-acetyl-1-benzylquinolinium, 6-acetyl-1-benzylquinolinium, 7-acetyl-1-benzylquinolinium, 8-acetyl-1-benzylquinolinium, 5-acetyl-1-allylquinolinium, 6-acetyl-1-allylquinolinium, 7-acetyl-1-allylquinolinium and 8-acetyl-1-allylquinolinium, 9-formyl-10-methylacridinium, 4-(2'-formylvinyl)-1-methylpyridinium, 1,3-dimethyl-2-(4'-formylphenyl)-benzimidazolium, 1,3-dimethyl-2-(4'-formylphenyl)-imidazolinium, 2-(4'-formylphenyl)-3-methylbenzothiazolium, 2-(4'-acetylphenyl)-3-methylbenzothiazolium, 2-(4'-formylphenyl)-3-methylbenzoxazolium, 2-(5'-formyl-2'-furyl)-3-methylbenzothiazolium, 2-(5'-formyl-2'-furyl)-3-methylbenzothiazolium, 2-(5'-formyl-2'-thienyl)-3-methylbenzothiazolium, 2-(3'-formylphenyl)-3-methylbenzothiazolium, 2-(4'-formyl-1-naphthyl)-3-methylbenzothiazolium, 5-chloro-2-(4'-formylphenyl)-3-methylbenzothiazolium, 2-(4'-formylphenyl)-3,5-dimethylbenzothiazolium benzenesulfonate, p-toluenesulfonate, methanesulfonate, perchlorate, sulfate, chloride, bromide, iodide, tetrachlorozincate, methylsulfate, trifluoromethanesulfonate, tetrafluoroborate, isatin, 1-methylisatin, 1-allylisatin, 1-hydroxymethylisatin, 5-chloroisatin, 5-methoxyisatin, 5-nitroisatin, 6-nitroisatin, 5-sulfoisatin, 5-carboxyisatin, quinisatin, 1-methylquinisatin, as well as any desired mixtures of the above-stated compounds.

7. An agent according to any one of claims 1 to 6, **characterised in that** component B is selected from the group consisting of vanillin, coniferyl aldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-3-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 4-hydroxy-3,5-dimethoxybenzaldehyde, 4-hydroxy-3,5-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaldehyde, 3,4-dihydroxy-6-methylbenzaldehyde, 3,4-dihydroxy-2-methoxybenzaldehyde, 3,4-dihydroxy-5-methoxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 3,4,5-trihydroxybenzaldehyde, 4-hydroxy-2-methoxybenzaldehyde, 4-dimethylaminobenzaldehyde, 4-diethylaminobenzaldehyde, 4-dimethylamino-2-hydroxybenzaldehyde, 4-diethylamino-2-hydroxybenzaldehyde, 4-pyrrolidinobenzaldehyde, 4-morpholinobenzaldehyde, 2-morpholinobenzaldehyde, 4-piperidinobenzaldehyde, 2-methoxy-1-naphthaldehyde, 4-methoxy-1-naphthaldehyde, 2-hydroxy-1-naphthaldehyde, 2,4-dihydroxy-1-napthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-hydroxy-1-naphthaldehyde, 4-dimethylamino-1-naphthaldehyde, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 4-dimethylamino-2-methoxybenzaldehyde, 4-(1-imidazolyl)-benzaldehyde and piperonal.

8. An agent according to any one of claims 1 to 7, **characterised in that** it contains as component C at least one compound selected from (a) CH-acidic compounds and (b) compounds with a primary or secondary amino or hydroxyl group, selected from aromatic hydroxyl compounds, primary or secondary aromatic amines and nitrogenous heterocyclic compounds.

9. An agent according to claim 9, **characterised in that** the component C CH-acidic compounds are selected from the group consisting of 1,2,3,3-tetramethyl-3H-indolium iodide, 1,2,3,3-tetramethyl-3H-indolium p-toluenesulfonate, 1,2,3,3-tetramethyl-3H-indolium methanesulfonate, 1,3,3-trimethyl-2-methyleneindoline (Fischer's base), 2,3-dimethylbenzothiazolium iodide, 2,3-dimethylbenzothiazolium p-toluenesulfonate, 2,3-dimethylnaphtho[1,2-d]thiazolium p-toluenesulfonate, 3-ethyl-2-methylnaphtho[1,2-d]thiazolium p-toluenesulfonate, rhodanine, rhodanine-3-acetic acid, 1,4-dimethylquinolinium iodide, 1,2-dimethylquinolinium iodide, barbituric acid, thiobarbituric acid, 1,3-dimethylthiobarbituric acid, 1,3-diethylthiobarbituric acid, 1,3-diethylbarbituric acid, oxindole, 3-indoxyl acetate, 2-coumaranone, 5-hydroxy-2-coumaranone, 6-hydroxy-2-coumaranone, 3-methyl-1-phenylpyrazolin-5-one, indan-1,2-dione, indan-1,3-dione, indan-1-one, benzoylacetonitrile, 3-dicyanomethyleneindan-1-one, 2-amino-4-imino-1,3-thiazoline hydrochloride, 5,5-dimethylcyclohexane-1,3-dione, 2H-1,4-benzoxazin-4H-3-one, 3-ethyl-2-methylbenzoxazolium iodide, 3-ethyl-2-methylbenzothiazolium iodide, 1-ethyl-4-methylquinolinium iodide, 1-ethyl-2-methylquinolinium iodide, 1,2,3-trimethylquinoxalinium iodide, 3-ethyl-2-methylbenzoxazolium p-toluenesulfonate, 3-ethyl-2-methylbenzothiazolium p-toluenesulfonate, 1-ethyl-4-methylquinolinium p-toluenesulfonate, 1-ethyl-2-methylquinolinium p-toluenesulfonate, 1,2,3-trimethylquinoxalinium p-toluenesulfonate, 1,2-dihydro-1,3,4,6-tetramethyl-2-oxopyrimidinium chloride, 1,2-dihydro-1,3,4,6-tetramethyl-2-oxopyrimidinium hydrogensulfate, 1,2-dihydro-1,3,4-trimethyl-2-thioxopyrimidinium chloride, 1,2-dihydro-4,6-dimethyl-1,3-dipropyl-2-oxopyrimidinium chloride, 1,2-dihydro-1,3,4,6-tetramethyl-2-thioxopyrimidinium hydrogensulfate and 2-dihydro-1,3,4,5,6-pentamethyl-2-oxopyrimidinium chloride.

10. An agent according to claim 8, **characterised in that** the component C primary and secondary aromatic amines are selected from the group consisting of N,N-dimethyl-p-phenylenediamine, N,N-diethyl-p-phenylenediamine, N-(2-hydroxyethyl)-N-ethyl-p-phenylenediamine, N,N-bis-(2'-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholinoaniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diaminophenetol, 4-amino-3-methylphenol, 2-(2,5-diaminophenyl)ethanol, 2,4-diaminophenoxyethanol, 2-(2,5-diamino-phenoxy)ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxy-ethylamino)-4-methoxy-2-methylphenol, 4-amino-2-hydroxymethyl-phenol, 2-(diethylaminomethyl)-4-aminophenol, 4-amino-1-hydroxy-2-(2-hydroxyethylaminomethyl)-benzene, 1-hydroxy-2-amino-5-methylbenzene, 1-hydroxy-2-amino-6-methylbenzene, 2-amino-5-acetamido-phenol, 1,3-dimethyl-2,5-diaminobenzene, 5-(3-hydroxypropylamino-)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy)-toluene, 2,4-diamino-5-methylphenetol, 3,5-diamino-2-methoxy-1-methylbenzene, 2-amino-4-(2'-hydroxyethylamino)-anisole, 2,5-bis-(2'-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulfonic acid, 3-aminobenzenesulfonic acid, 4-aminobenzenesulfonic acid, 3-amino-4-hydroxybenzenesulfonic acid, 4-amino-3-hydroxynaphthalene-1-sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechol, 4,6-diaminopyrogallol, 1-(2-hydroxy-5-aminobenzyl)-2-imidazolidinone, 4-amino-2-((4-[(5-amino-2-hydroxyphenyl)methyl]-piperazinyl)methyl)phenol, 3,5-diamino-4-hydroxypyrocatechol, 1,4-bis-(4-aminophenol)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, nitro group-containing amino compounds, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo]-7-hydroxynaphth-2-yl]trimethylammonium chloride (Basic Brown 17), 1-hydroxy-2-amino-4,6-dinitrobenzene, 1-amino-2-nitro-4-[bis-(2-hydroxyethyl)amino]benzene, 1-amino-2-(2-hydroxyethyl)-amino-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)-amino]-2-nitro-4-aminobenzene (HC Red No. 3), 4-amino-3-nitrophenol, 4-amino-2-nitrophenol, 6-nitro-o-toluidine, 1-amino-3-methyl-4-[(2-hydroxyethyl)-amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)-benzoic acid, 6-nitro-2,5-diaminopyrimidine, 2-amino-6-chloro-4-nitrophenol, 1-amino-2-(3-nitrophenylazo)-7-phenylazo-8-naphthol-3,6-disulfonic acid disodium salt (Acid Blue No. 29), 1-amino-2-(2-hydroxy-4-nitrophenylazo)-8-naphthol-3,6-disulfonic acid disodium salt (Palatinchrome Green), 1-amino-2-(3-chloro-2-hydroxy-5-nitrophenylazo)-8-naphthol-3,6-disulfonic acid disodium salt (Gallion), 4-amino-4'-nitrostilbene-2,2'-disulfonic acid disodium salt, 2,4-diamino-3',5'-dinitro-2'-hydroxy-5-methylazobenzene (Mordant Brown 4), 4'-amino-4-nitrodiphenylamine-2-sulfonic acid, 4'-amino-3'-nitrobenzophenone-2-carboxylic acid, 1-amino-4-nitro-2-(2-nitrobenzylideneamino)-benzene, 2-[2-(diethylamino)ethylamino]-5-nitroaniline, 3-amino-4-hydroxy-5-nitrobenzenesulfonic acid, 3-amino-3'-nitrobiphenyl, 3-amino-4-nitro-acenaphthene, 2-amino-1-nitronaphthalene, 5-amino-6-nitrobenzo-1,3-dioxole, anilines, in particular nitro group-containing anilines, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis-(2'-hydroxyethyl)-amino]-benzene, 4-amino-2-nitrodiphenylamine-2'-carboxylic acid, 1-amino-5-chloro-4-(2-hydroxyethyl-amino)-2-nitrobenzene, aromatic anilines or phenols with a further aromatic residue, as are shown in the formula VII 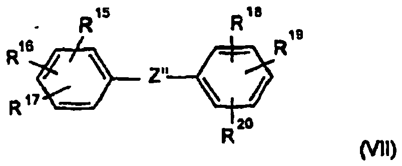 in which
• R¹⁵ denotes a hydroxyl or an amino group, which may be substituted by C₁-₆ alkyl, C₁-C₆ hydroxyalkyl, C₁-C₆ alkoxy or C₁-C₆-alkoxy-C₁-C₆-alkyl groups,
• R¹⁶, R¹⁷, R¹⁸, R¹⁹ and R²⁰ mutually independently denote a hydrogen atom, a hydroxyl or an amino group which may be substituted by C₁-C₆ alkyl, C₁-C₆ hydroxyalkyl, C₁-C₆ alkoxy, C₁-C₆ aminoalkyl or C₁-C₆-alkoxy-C₁-C₆-alkyl groups, and
• Z" denotes a direct bond, a saturated or unsaturated carbon chain with 1 to 4 carbon atoms optionally substituted by hydroxyl groups, a carbonyl, sulfoxy, sulfonyl or imino group, an oxygen or sulfur atom, or a group of the formula VIII
-Q'(_{̅}CH₂-Q-CH₂-Q")_{̅o} (VIII)
in which
• Q means a direct bond, a CH₂ or CHOH group,
• Q' and Q" mutually independently denote an oxygen atom, an NR²¹ group, in which R²¹ means a hydrogen atom, a C₁-C₆ alkyl group or C₁-C₆ hydroxyalkyl group, wherein the two groups, together with the remainder of the molecule, may form a 5-, 6- or 7-membered ring, the group O-(CH₂)ₚ-NH or NH-(CH₂)_{p'}-O, in which p and p' are 2 or 3 and
• o means a number from 1 to 4,
such as in particular 4,4'-diaminostilbene and the hydrochloride thereof, 4,4'-diaminostilbene-2,2'-disulfonic acid mono- or disodium salt, 4-amino-4'-dimethylaminostilbene and the hydrochloride thereof, 4,4'-diaminodiphenylmethane, 4,4'-diaminodiphenyl sulfide, 4,4'-diaminodiphenyl sulfoxide, 4,4'-diaminodiphenylamine, 4,4'-diaminodiphenylamine-2-sulfonic acid, 4,4'-diaminobenzophenone, 4,4'-diaminodiphenyl ether, 3,3',4,4'-tetraaminodiphenyl, 3,3',4,4'-tetraaminobenzophenone, 1,3-bis-(2,4-diaminophenoxy)propane, 1,8-bis-(2,5-diaminophenoxy)-3,6-dioxaoctane, 1,3-bis-(4-aminophenylamino)propane, 1,3-bis-(4-aminophenylamino)-2-propanol, 1,3-bis-[N-(4-aminophenyl)-2-hydroxyethylamino]-2-propanol, N,N-bis-[2-(4-aminophenoxy)-ethyl]methylamine, N-phenyl-1,4-phenylenediamine and bis-(5-amino-2-hydroxyphenyl)-methane.

11. An agent according to claim 8, **characterised in that** the component C aromatic hydroxyl compounds are selected from the group consisting of 2-methylresorcinol, 4-methylresorcinol, 5-methylresorcinol, 2,5-dimethyl-resorcinol, resorcinol, 3-methoxyphenol, pyrocatechol, hydroquinone, pyrogallol, phloroglucinol, hydroxyhydroquinone, 2-methoxyphenol, 3-methoxyphenol, 4-methoxyphenol, 3-dimethylaminophenol, 2-(2-hydroxyethyl)phenol, 3,4-methylenedioxyphenol, 2,4-dihydroxybenzoic acid, 3,4-dihydroxybenzoic acid, 1-(2,4-dihydroxyphenyl)acetic acid, 1-(3,4-dihydroxyphenyl)acetic acid, gallic acid, 2,4,6-trihydroxybenzoic acid, -acetophenone, 2-chlororesorcinol, 4-chlororesorcinol, 1-naphthol, 1,5-dihydroxynaphthalene, 2,3-dihydroxynaphthalene, 2,7-dihydroxynaphthalene, 6-dimethylamino-4-hydroxy-2-naphthalenesulfonic acid and 3,6-dihydroxy-2,7-naphthalenesulfonic acid.

12. An agent according to claim 8, **characterised in that** the component C nitrogenous heterocyclic compounds are selected from the group consisting of 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyrimidine, 2,4,5-triaminopyridine, 2,6-dihydroxy-3,4-dimethylpyridine, N-[2-(2,4-diaminophenyl)aminoethyl]-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4,5,6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrine), 1-phenyl-3-methylpyrazol-5-one, 2-aminoquinoline, 3-aminoquinoline, 8-aminoquinoline, 4-aminoquinaldine, 2-aminonicotinic acid, 6-aminonicotinic acid, 5-aminoisoquinoline, 5-aminoindazole, 6-aminoindazole, 5-aminobenzimidazole, 7-aminobenzimidazole, 5-aminobenzothiazole, 7-aminobenzothiazole, 2,5-dihydroxy-4-morpholinoaniline together with indole and indoline derivatives, such as 4-aminoindole, 5-aminoindole, 6-aminoindole, 7-aminoindole, 5,6-dihydroxyindole, 5,6-dihydroxyindoline, 4-hydroxyindoline and hydroxypyrimidine derivatives and the physiologically acceptable salts of the above-stated compounds.

13. An agent according to any one of claims 1 to 12, **characterised in that** the compounds of the formula I, the component B compounds and the component C compounds are in each case present in a quantity of 0.03 to 65 mmol, in particular of 1 to 40 mmol, relative to 100 g of the total dyeing agent.

14. An agent according to any one of claims 1 to 13, **characterised in that** it contains at least one reaction product RP according to formula IX 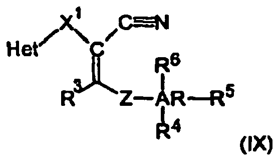 in which Het, X¹, R³, R⁴, R⁵, R⁶, AR and Z are defined as in claim 5,
with the proviso that, if R³ denotes a hydrogen atom and Z a direct bond and the grouping -[ArR⁴R⁵R⁶] of the formula IX denotes a 3,5-dichloro-4-hydroxyphenyl group, Het is not derived from benzofuran, thiazole or benzothiazole.

15. An agent according to claim 14, **characterised in that** AR according to the formula IX denotes benzene or naphthalene.

16. An agent according to either one of claims 14 to 15, **characterised in that** Z according to the formula IX denotes a direct bond or vinylene.

17. An agent according to any one of claims 14 to 16, **characterised in that** R³ according to the formula IX denotes a hydrogen atom.

18. An agent according to any one of claims 14 to 17, **characterised in that** the reaction product RP is present in a quantity of 0.03 to 65 mmol, in particular of 1 to 40 mmol, relative to 100 g of the entire dyeing agent.

19. An agent according to any one of claims 1 to 18, **characterised in that** it contains colour enhancers selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, arginine, histidine, pyrrolidine, proline, pyrrolidone, pyrrolidone-5-carboxylic acid, pyrazole, 1,2,4-triazole, piperazidine or any desired mixtures thereof.

20. An agent according to any one of claims 1 to 19, **characterised in that** it contains at least one direct dye, preferably in a quantity of from 0.01 to 20 wt.%, relative to the total dyeing agent.

21. An agent according to any one of claims 1 to 20, **characterised in that** it contains at least one developer component and optionally at least one coupler component as oxidation dye precursor.

22. An agent according to any one of claims 1 to 21, **characterised in that** ammonium or metal salts are added which are selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc.

23. An agent according to any one of claims 1 to 22, **characterised in that** it contains oxidising agents, in particular H₂O₂, in a quantity of 0.01 to 6 wt.%, relative to the application solution.

24. An agent according to any one of claims 1 to 23, **characterised in that** it contains anionic, zwitterionic or nonionic surfactants.

25. Use of at least one compound of the formula I according to claim 1, in combination with at least one reactive carbonyl compound, as a dyeing component in hair dyeing agents.

26. A method for dyeing keratin-containing fibres, in particular human hair, in which a dyeing agent according to claim 1 together with conventional cosmetic ingredients are applied onto the keratin-containing fibres, left on the fibres for a period of time, conventionally approx. 15-30 minutes, and then rinsed out again or washed out with a shampoo.

27. A method according to claim 26, **characterised in that**, in a two-stage method, component B is applied onto the keratin-containing fibres before or after application of the compound according to the formula I, the mixture obtained on the hair is left on the fibres for a period of time, conventionally approx. 15-30 minutes, and then rinsed out again or washed out with a shampoo.

28. A method according to either one of claims 26 or 27, **characterised in that**, for the purposes of pretreatment, before a dyeing agent according to claim 1 is applied, the keratin-containing fibres were blonded with a blonding agent or dyed with an oxidation dyeing agent.

29. Use of at least one compound according to the formula I, 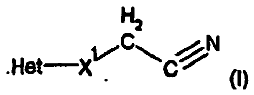 wherein Het and X¹ are defined as in claim 1, in combination with reactive carbonyl compounds (component B), for shading oxidation dyed keratin-containing fibres, in particular human hair.

30. Use of at least one compound according to the formula I, 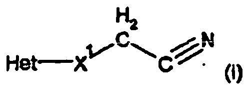 wherein Het and X¹ are defined as in claim 1, in combination with reactive carbonyl compounds (component B), for freshening up the colour of keratin-containing fibres dyed with oxidative dyeing agents.

## Revendications

1. Agent pour la teinture de fibres kératiniques, en particulier de cheveux, contenant, dans un support cosmétique, aqueux, comme composant A, au moins un composé selon la formule I ou son sel physiologiquement acceptable, 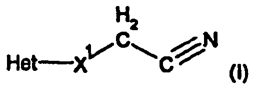 où
- Het représente un hétéroaromatique le cas échéant substitué,
- X¹ représente une liaison directe ou un groupe carbonyle,
et comme composant B au moins un composé présentant un groupe carbonyle réactif.

2. Agent selon la revendication 1, **caractérisé en ce que** le radical Het représente 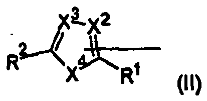 où
R¹ et R² représentent, indépendamment l'un de l'autre, un atome d'hydrogène, un groupe hydroxy, un atome d'halogène, un groupe nitro, un groupe C₁-C₆-alkyle linéaire ou cyclique, un groupe C₂-C₆-alcényle, un groupe aryle le cas échéant substitué, un groupe cyanométhyle, un groupe cyanométhylcarbonyle, un groupe hétéroaryle le cas échéant substitué, un groupe aryl-C₁-C₆-alkyle, un groupe C₁-C₆-hydroxyalkyle, un groupe C₂-C₆-polyhydroxyalkyle, un groupe C₁-C₆-alcoxy, un groupe C₁-C₆-alcoxycarbonyle, un groupe C₁-C₆-alcoxy-C₂-C₆-alkyle, un groupe C₁-C₆-sulfoalkyle, un groupe C₁-C₆-carboxyalkyle, un groupe R^{I}R^{II}N-(CH₂)ₘ-, R^{I} et R^{II} représentant, indépendamment l'un de l'autre, un atome d'hydrogène, un groupe C₁-C₆-alkyle linéaire ou cyclique, un groupe C₂-C₆-alcényle, un groupe C₁-C₆-hydroxyalkyle ou un groupe aryl-C₁-C₄-alkyle, R^{I} et R^{II} pouvant former, ensemble avec l'atome d'azote, un cycle de 5, 6 ou 7 chaînons et m représentant un nombre 0, 1, 2, 3 ou 4, R¹ et/ou R² pouvant former un cycle de 5 ou 6 chaînons aromatique ou hétéroaromatique, le cas échéant substitué, condensé sur le cycle du reste de la molécule
X² et X³ représentent, indépendamment l'un de l'autre, un atome d'azote ou un groupe CR³, R³ représentant un atome d'hydrogène, un groupe hydroxy, un atome d'halogène, un groupe nitro, un groupe C₁-C₆-alkyle linéaire ou cyclique, un groupe C₂-C₆-alcényle, un groupe aryle le cas échéant substitué, un groupe cyanométhyle, un groupe cyanométhylcarbonyle, un groupe hétéroaryle le cas échéant substitué, un groupe aryl-C₁-C₆-alkyle, un groupe C₁-C₆-hydroxyalkyle, un groupe C₂-C₆-_{P}olyhydroxyalkyle, un groupe C₁-C₆-alcoxy, un groupe C₁-C₆-alcoxycarbonyle, un groupe C₁-C₆-alcoxy-C₂-C₆-alkyle, un groupe C₁-C₆-sulfoalkyle, un groupe C₁-C₆-carboxyalkyle et un groupe R^{III}R^{IV}N-(CH₂)ₙ-, R^{III} et R^{IV} représentant, indépendamment l'un de l'autre, un atome d'hydrogène, un groupe C₁-C₆-alkyle linéaire ou cyclique, un groupe C₂-C₆-alcényle, un groupe C₁-C₆-hydroxyalkyle ou un groupe aryl-C₁-C₄-alkyle, R^{III} et R^{IV} pouvant former, ensemble avec l'atome d'azote, un cycle de 5, 6 ou 7 chaînons et n représentant un nombre 0, 1, 2, 3 ou 4, au moins un des substituants X² et X³ pouvant former, ensemble avec le reste de la molécule, un cycle de 5 ou 6 chaînons, aromatique, le cas échéant substitué, condensé,
X⁴ représente un atome d'oxygène, un atome de soufre, un groupe vinylène ou un groupe N-H, les deux derniers groupes mentionnés pouvant être substitués, indépendamment l'un de l'autre, par un groupe C₁-C₆-alkyle linéaire ou cyclique, un groupe C₂-C₆-alcényle, un groupe aryle le cas échéant substitué, un groupe hétéroaryle le cas échéant substitué, un groupe aryl-C₁-C₆-alkyle, un groupe C₂-C₆-hydroxyalkyle, un groupe C₂-C₆-polyhydroxyalkyle, un groupe C₁-C₆-alcoxy-C₂-C₆-alkyle, un groupe C₁-C₆-sulfoalkyle, un groupe C₁-C₆-carboxyalkyle, un groupe R^{V}R^{VI}N-(CH₂)ₚ-, R^{V} et R^{VI} représentant, indépendamment l'un de l'autre, un atome d'hydrogène, un groupe C₁-C₆-alkyle linéaire ou cyclique, un groupe C₂-C₆-alcényle, un groupe C₁-C₆-hydroxyalkyle ou un groupe aryl-C₁-C₄-alkyle, R^{V} et R^{VI} pouvant former, ensemble avec l'atome d'azote, un cycle de 5, 6 ou 7 chaînons et p représentant un nombre 0, 1, 2, 3 ou 4,
à condition que lorsque X⁴ représente un groupe vinylène, au moins un des groupes X² ou X³ signifie un atome d'azote.

3. Agent selon l'une quelconque des revendications 1 à 2, **caractérisé en ce que** le radical Het est dérivé d'un hétéroaromatique choisi parmi le furanne, le thiophène, le pyrrole, l'isoxazole, l'isothiazole, l'imidazole, l'oxazole, le thiazole, la pyridine, la pyridazine, la pyrimidine, la pyrazine, la 1,2,3-triazine, la 1,2,4-triazine, la 1,3,5-triazine, le benzopyrrole, le benzofuranne, le benzothiophène, le benzimidazole, le benzoxazole, l'indazole, le benzoisoxazole, le benzo-isothiazole, l'indole, la quinoléine, l'isoquinoléine, la cinnoline, la phtalazine, la quinazoline, la quinoxaline, l'acridine, la benzoquinoléine, la benzo-isoquinoléine, la phénazine, la benzocinnoline, la benzoquinazoline, la benzoquinoxaline, la phénoxazine, la phénothiazine, la néphtyridine, la phénanthroline, l'indolizine, la quinolizine, la carboline, la purine, la ptéridine et la coumarine, les hétéroaromatiques susmentionnés pouvant être substitués par au moins un groupe choisi parmi un atome d'halogène, un groupe nitro, un groupe thio, un groupe thio-(C₁-C₆)-alkyle, un groupe hétéroaryle, un groupe aryle, un groupe (C₁-C₆)-alkyle, un groupe (C₁-C₆)-alcoxy, un groupe hydroxy, un groupe (C₂-C₆)-hydroxyalkyle, un groupe (C₂-C₆)-polyhydroxyalkyle, un groupe (C₁-C₆)-alcoxyl-(C₁-C₆)-alkyle, un groupe aryl-(C₁-C₆)-alkyle, un groupe amino, un groupe (C₁-C₆)-monoalkylamino, un groupe (C₁-C₆)-dialkylamino, un groupe dialkylaminoalkyle -(CH₂)ₙ-NR'R", n représentant un nombre entier de 2 et 6 et R' et R" signifiant, indépendamment l'un de l'autre, un groupe alkyle linéaire ou ramifié, qui peuvent le cas échéant former ensemble un cycle.

4. Agent selon l'une quelconque des revendications 1 à 3, **caractérisé en ce que** les composés selon la formule I sont choisis dans le groupe constitué par le 2-(5-bromo-2-furoyl)-acétonitrile, le 2-(5-méthyl-2-trifluorométhyl-3-furoyl)-acétonitrile, le 3-(2,5-diméthyl-3-furyl)-3-oxopropanitrile, le 2-(2-thénoyl)-acétonitrile, le 2-(3-thénoyl)-acétonitrile, le 2-(5-fluoro-2-thénoyl)-acétonitrile, le 2-(5-chloro-2-thénoyl)-acétonitrile, le 2-(5-bromo-2-thénoyl)-acétonitrile, le 2-(5-méthyl-2-thénoyl)-acétonitrile, le 2-(2,5-diméthylpyrrol-3-oyl)-acétonitrile, le 2-(1,2,5-triméthylpyrrol-3-oyl)-acétonitrile, le 1H-benzimidazol-2-ylacétonitrile, le 1 H-benzothiazol-2-ylacétonitrile, le 2-(pyrid-2-yl)-acétonitrile, le 2,6-bis(cyanométhyl)-pyridine, le 2-(indol-3-oyl)-acétonitrile, le 2-(2-méthylindol-3-oyl)-acétonitrile, la 8-cyanoacétyl-7-méthoxy-4-méthylcoumarine, le 2-(2-isopropyl-5,6-benzoquinoléin-4-oyl)-acétonitrile, le 2-(2-phényl-5,6-benzoquinoléin-4-oyl)-acétonitrile, le 2-(quinoxalin-2-yl)-acétonitrile, le 2-(coumaron-2-yl)-acétonitrile, l'ester tert-butylique de l'acide 6,7-dichloro-5-(cyanoacétyl)-2,3-dihydro-1-benzofuranne-2-carboxylique, le 2-(6-hydroxy-4,7-diméthoxy-1-benzofurann-5-oyl)-acétonitrile et le 2-(1-phényl-1,4-dihydrothiochroméno[4,3-c]pyrazol-3-oyl)-acétonitrile.

5. Agent selon l'une quelconque des revendications 1 à 4, **caractérisé en ce que** le composant B est choisi parmi un composé selon la formule III 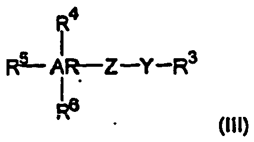 où
AR représente benzène, naphtalène, pyridine, pyrimidine, pyrazine, pyridazine, carbazole, pyrrole, pyrazole, furanne, thiophène, 1,2,3-triazine, 1,3,5-triazine, quinoléine, isoquinoléine, indole, indoline, indolizine, indane, imidazole, 1,2,4-triazole, 1,2,3-triazole, tétrazole, benzimidazole, 1,3-thiazole, benzothiazole, indazole, benzoxazole, quinoxaline, quinazoline, quinolizine, cinnoline, acridine, julolidine, acénaphtène, fluorène, biphényle, diphénylméthane, benzophénone, diphényléther, azobenzène, chromone, coumarine, diphénylamine, stilbène, les N-hétéroaromatiques pouvant également être quaternisés,
R³ représente un atome d'hydrogène, un groupe C₁-C₆-alkyle, C₂-C₆-acyle, C₂-C₆-alcényle, C₁-C₄-perfluoroalkyle, un groupe aryle ou hétéroaryle le cas échéant substitué,
R⁴, R⁵ et R⁶ représentent, indépendamment l'un de l'autre, un atome d'hydrogène, un atome d'halogène, un groupe C₁-C₆-alkyle, C₁-C₆-alcoxy, C₁-C₆-aminoalkyle, C₁-C₆-hydroxyalkyle, un groupe C₁-C₆-alcoxy-C₁-C₆-alkyloxy, un groupe C₂-C₆-acyle, un groupe acétyle, carboxyle, carboxylato, carbamoyle, sulfo, sulfato, sulfonamide, sulfonamido, C₂-C₆-alcényle, aryle, aryl-C₁-C₆-alkyle, un groupe hydroxy, un groupe nitro, un groupe pyrrolidino, un groupe morpholino, un groupe pipéridino, un groupe amino ou, selon le cas, ammonio ou un groupe 1-imidazol(in)io, les trois derniers groupes mentionnés pouvant être substitués par un ou plusieurs groupes C₁-C₆-alkyle, C₁-C₆-carboxyalkyle, C₁-C₆-hydroxyalkyle, C₂-C₆-alcényle, C₁-C₆-alcoxy-C₁-C₆-alkyle, benzyle le cas échéant substitués, sulfo-(C₁-C₄)-alkyle ou hétérocycle-(C₁-C₄)-alkyle, deux des radicaux parmi R⁴, R⁵, R⁶ et -Z-Y-R³ pouvant également former, ensemble avec la molécule résiduelle, un cycle de 5, 6 ou 7 chaînons, condensé sur le reste de la molécule, le cas échéant substitué, qui peut également porter un cycle aromatique condensé, le système AR pouvant porter, en fonction de la taille du cycle, d'autres substituants, qui peuvent représenter indépendamment l'un de l'autre, les mêmes groupes que R⁴, R⁵ et R⁶,
Z représente une liaison directe, un groupe carbonyle, carboxy-(C₁-C₄)-alkylène, un groupe C₂-C₆-alcénylène le cas échéant substitué, C₄-C₆-alcadiénylène, furylène, thiénylène, arylène, vinylènearylène, vinylènefurylène, vinylène-thiénylène, Z pouvant également former, ensemble avec le groupe -Y-R³, un cycle de 5, 6 ou 7 chaînons, le cas échéant substitué,
Y représente un groupe qui est choisi parmi carbonyle, un groupe selon la formule IV et un groupe selon la formule V, 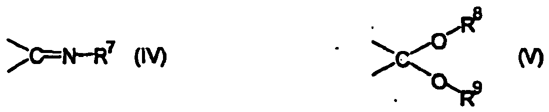 où
R⁷ représente un atome d'hydrogène, un groupe hydroxy, un groupe C₁-C₄-alcoxy, un groupe C₁-C₆-alkyle, un groupe C₁-C₆-hydroxyalkyle, un groupe C₂-C₆-polyhydroxyalkyle, un groupe C₁-C₆-alcoxy-C₁-C₆-alkyle,
R⁸ et R⁹ représentent, indépendamment l'un de l'autre, un atome d'hydrogène, un groupe C₁-C₆-alkyle, un groupe aryle ou forment ensemble avec l'élément de structure O-C-O de la formule V un cycle de 5 ou 6 chaînons.

6. Agent selon l'une quelconque des revendications 1 à 5, **caractérisé en ce que** le composant B est choisi dans le groupe constitué par l'acétophénone, la propiophénone, la 2-hydroxyacétophénone, la 3-hydroxyacétophénone, la 4-hydroxyacétophénone, la 2-hydroxypropiophénone, la 3-hydroxypropiophénone, la 4-hydroxypropiophénone, la 2-hydroxybutyrophénone, la 3-hydroxybutyrophénone, la 4-hydroxybutyrophénone, la 2,4-dihydroxyacétophénone, la 2,5-dihydroxyacétophénone, la 2,6-dihydroxyacétophénone, la 2,3,4-trihydroxyacétophénone, la 3,4,5-trihydroxyacétophénone, la 2,4,6-trihydroxyacétophénone, la 2,4,6-triméthoxyacétophénone, la 3,4,5-triméthoxyacétophénone, le 3,4,5-triméthoxyacétophénonediéthylcétal, la 4-hydroxy-3-méthoxyacétophénone, la 3,5-diméthoxy-4-hydroxyacétophénone, la 4-aminoacétophénone, la 4-diméthylaminoacétophénone, la 4-morpholinoacétophénone, la 4-pipéridinoacétophénone, la 4-imidazolinoacétophénone, la 2-hydroxy-5-bromoacétophénone, la 4-hydroxy-3-nitroacétophénone, l'acide acétophénone-2-carboxylique, l'acide acétophénone-4-carboxylique, la benzophénone, la 4-hydroxybenzophénone, la 2-aminobenzophénone, la 4,4'-dihydroxybenzophénone, la 2,4-dihydroxybenzophénone, la 2,4,4'-trihydroxybenzophénone, la 2,3,4-trihydroxybenzophénone, la 2-hydroxy-1-acétonaphtone, la 1-hydroxy-2-acétonaphtone, la chromone, l'acide chromone-2-carboxylique, la flavone, la 3-hydroxyflavone, la 3,5,7-trihydroxyflavone, la 4,5,7-trihydroxyflavone, la 5,6,7-trihydroxyflavone, la quercétine, la 1-indanone, la 9-fluorénone, la 3-hydroxyfluorénone, l'anthrone, la 1,8-dihydroxyanthrone, la vanilline, le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxybenzaldéhyde, le 4-hydroxy-2,5-diméthoxybenzaldéhyde, le 4-hydroxy-2,6-diméthoxybenzaldéhyde, le 4-hydroxy-2-méthylbenzaldéhyde, le 4-hydroxy-3-méthylbenzaldéhyde, le 4-hydroxy-2,3-diméthylbenzaldéhyde, le 4-hydroxy-2,5-diméthylbenzaldéhyde, le 4-hydroxy-2,6-diméthylbenzaldéhyde, le 4-hydroxy-3,5-diméthoxybenzaldéhyde, le 4-hydroxy-3,5-diméthylbenzaldéhyde, le 3,5-diéthoxy-4-hydroxybenzaldéhyde, le 2,6-diéthoxy-4-hydroxybenzaldéhyde, le 3-hydroxy-4-méthoxybenzaldéhyde, le 2-hydroxy-4-méthoxybenzaldéhyde, le 2-éthoxy-4-hydroxybenzaldéhyde, le 3-éthoxy-4-hydroxybenzaldéhyde, le 4-éthoxy-2-hydroxybenzaldéhyde, le 4-éthoxy-3-hydroxybenzaldéhyde, le 2,3-diméthoxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,5-diméthoxybenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,4-dihydroxy-3-méthylbenzaldéhyde, le 2,4-dihydroxy-5-méthylbenzaldéhyde, le 2,4-dihydroxy-6-méthylbenzaldéhyde, le 2,4-dihydroxy-3-méthoxybenzaldéhyde, le 2,4-dihydroxy-5-méthoxybenzaldéhyde, le 2,4-dihydroxy-6-méthoxybenzaldéhyde, le 2,5-dihydroxybenzaldéhyde, le 2,6-dihydroxybenzaldéhyde, le 3,4-dihydroxybenzaldéhyde, le 3,4-dihydroxy-2-méthylbenzaldéhyde, le 3,4-dihydroxy-5-méthylbenzaldéhyde, le 3,4-dihydroxy-6-méthylbenzaldéhyde, le 3,4-dihydroxy-2-méthoxybenzaldéhyde, le 3,4-dihydroxy-5-méthoxybenzaldéhyde, le 3,5-dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 3,4,5-trihydroxybenzaldéhyde, le 2,5,6-trihydroxybenzaldéhyde, le 4-hydroxy-2-méthoxybenzaldéhyde, le 4-diméthylaminobenzaldéhyde, le 4-diéthylaminobenzaldéhyde, le 4-diméthylamino-2-hydroxybenzaldéhyde, le 4-diéthylamino-2-hydroxybenzaldéhyde, le 4-pyrrolidinobenzaldéhyde, le 4-morpholinobenzaldéhyde, le 2-morpholinobenzaldéhyde, le 4-pipéridinobenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-napthaldéhyde, le 4-hydroxy-3-méthoxy-1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-hydroxy-1-naphtaldéhyde, le 4-diméthylamino-1-naphtaldéhyde, l'aldéhyde 4-diméthylaminocinnamique, le 2-diméthylaminobenzaldéhyde, le 2-chloro-4-diméthylaminobenzaldéhyde, le 4-diméthylamino-2-méthylbenzaldéhyde, l'aldéhyde 4-diéthylaminocinnamique, le 4-dibutylaminobenzaldéhyde, le 4-diphénylaminobenzaldéhyde, le 4-diméthylamino-2-méthoxybenzaldéhyde, le 4-(1-imidazolyl)-benzaldéhyde, le pipéronal, le 2,3,6,7-tétrahydro-1H,5H-benzo[ij]quinolizine-9-carboxaldéhyde, le 2,3,6,7-tétrahydro-8-hydroxy-1H,5H-benzo[ij]quinolizine-9-carboxaldéhyde, le N-éthylcarbazol-3-aldéhyde, la 2-formylméthylène-1,3,3-triméthylindoline (aldéhyde de Fischer ou aldéhyde tribasique), le 2-indolaldéhyde, le 3-indolaldéhyde, le 1-méthylindol-3-aldéhyde, le 2-méthylindol-3-aldéhyde, le 1-acétylindol-3-aldéhyde, le 3-acétylindole, le 1-méthyl-3-acétylindole, le 2-(1',3',3'-triméthyl-2-indolinylidène)-acétaldéhyde, le 1-méthylpyrrol-2-aldéhyde, le 1-méthyl-2-acétylpyrrole, le 4-pyridinaldéhyde, le 2-pyridinaldéhyde, le 3-pyridinaldéhyde, la 4-acétylpyridine, la 2-acétylpyridine, la 3-acétylpyridine, le pyridoxal, le quinoléin-3-aldéhyde, le quinoléin-4-aldéhyde, l'antipyrin-4-aldéhyde, le furfural, le 5-nitrofurfural, la 2-thénoyltrifluoroacétone, le chromon-3-aldéhyde, la 3-(5'-nitro-2'-furyl)-acroléine, la 3-(2'-furyl)-acroléine et l'imidazol-2-aldéhyde, le 1,3-diacétylbenzène, le 1,4-diacétylbenzène, le 1,3,5-triacétylbenzène, la 2-benzoylacétophénone, la 2-(4'-méthoxybenzoyl)-acétophénone, la 2-(2'-furoyl)-acétophénone, la 2-(2'-pyridoyl)-acétophénone et la 2-(3'-pyridoyl)-acétophénone, la benzylidène-acétone, la 4-hydroxybenzylidène-acétone, la 2-hydroxybenzylidène-acétone, la 4-méthoxybenzylidène-acétone, la 4-hydroxy-3-méthoxybenzylidène-acétone, la 4-diméthylaminobenzylidène-acétone, la 3,4-méthylènedioxybenzylidène-acétone, la 4-pyrrolidinobenzylidène-acétone, la 4-pipéridinobenzylidène-acétone, la 4-morpholinobenzylidène-acétone, la 4-diéthylaminobenzylidène-acétone, la 3-benzylidène-2,4-pentanedione, la 3-(4'-hydroxybenzylidène)-2,4-pentanedione, la 3-(4'-diméthylaminobenzylidène)-2,4-pentanedione, la 2-benzylidènecyclohexanone, la 2-(4'-hydroxybenzylidène)-cyclohexanone, la 2-(4'-diméthylaminobenzylidène)-cyclohexanone, la 2-benzylidène-1,3-cyclohexanedione, la 2-(4'-hydroxybenzylidène)-1,3-cyclohexanedione, la 3-(4'-diméthylaminobenzylidène)-1,3-cyclohexanedione, la 2-benzylidène-5,5-diméthyl-1,3-cyclohexanedione, la 2-(4'-hydroxybenzylidène)-5,5-diméthyl-1,3-cyclohexanedione, la 2-(4'-hydroxy-3-méthoxybenzylidène)-5,5-diméthyl-1,3-cyclohexanedione, la 2-(4'-diméthylaminobenzylidène)-5,5-diméthyl-1,3-cyclohexanedione, la 2-benzylidènecyclopentanone, la 2'-(4-hydroxybenzylidène)-cyclopentanone, la 2-(4'-diméthylaminobenzylidène)-cyclopentanone, le 5-(4-diméthylaminophényl)penta-2,4-diénal, 5-(4-diéthylaminophényl)penta-2,4-diénal, le 5-(4-méthoxyphényl)penta-2,4-diénal, le 5-(3,4-diméthoxyphényl)penta-2,4-diénal, le 5-(2,4-diméthoxyphényl)penta-2,4-diénal, le 5-(4-pipéridinophényl)penta-2,4-diénal, le 5-(4-morpholinophényl)penta-2,4-diénal, le 5-(4-pyrrodinophényl)penta-2,4-diénal, la 6-(4-diméthylaminophényl)hexa-3,5-dién-2-one, la 6-(4-diéthylaminophényl)hexa-3,5-dién-2-one, la 6-(4-méthoxyphényl)hexa-3,5-dién-2-one, la 6-(3,4-diméthoxyphényl)hexa-3,5-dién-2-one, la 6-(2,4-diméthoxyphényl)hexa-3,5-dién-2-one, la 6-(4-pipéridinophényl)hexa-3,5-dién-2-one, la 6-(4-morpholinophényl)hexa-3,5-dién-2-one, la 6-(4-pyrrolidinophényl)hexa-3,5-dién-2-one, le 5-(4-diméthylamino-1-naphtyl)penta-3,5-diénal, le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 4-hydroxy-3-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5-nitrobenzaldéhyde, le 4,5-diméthoxy-2-nitrobenzaldéhyde, le 6-nitropipéronal, le 2-nitropipéronal, la 5-nitrovanilline, le 2,5-dinitrosalicylaldéhyde, le 5-bromo-3-nitrosalicylaldéhyde, l'acide 3-nitro-4-formylbenzènesulfonique, le 4-nitro-1-naphtaldéhyde, l'aldéhyde 2-nitrocinnamique, l'aldéhyde 3-nitrocinnamique, l'aldéhyde 4-nitrocinnamique, le 9-méthyl-3-carbazolaldéhyde, le 9-éthyl-3-carbazolaldéhyde, le 3-acétylcarbazole, le 3,6-diacétyl-9-éthylcarbazole, le 3-acétyl-9-méthylcarbazole, le 1,4-diméthyl-3-carbazolaldéhyde, le 1,4,9-triméthyl-3-carbazolaldéhyde, le benzènesulfonate, p-toluènesulfonate, méthanesulfonate, perchlorate, sulfate, chlorure, bromure, iodure, tetrachlorozinçate, méthylsulfate, trifluorométhanesulfonate, tétrafluoroborate de 4-formyl-1-méthylpyridinium, 2-formyl-1-méthylpyridinium, 4-formyl-1-éthylpyridinium, 2-formyl-1-éthylpyridinium, 4-formyl-1-benzylpyridinium, 2-formyl-1-benzylpyridinium, 4-formyl-1,2-diméthylpyridinium, 4-formyl-1,3-diméthylpyridinium, 4-formyl-1-méthylquinoléinium, 2-formyl-1-méthylquinoléinium, 4-acétyl-1-méthylpyridinium, 2-acétyl-1-méthylpyridinium, 4-acétyl-1-méthylquinoléinium, 5-formyl-1-méthylquinoléinium, 6-formyl-1-méthylquinoléinium, 7-formyl-1-méthylquinoléinium, 8-formyl-1-méthylquinoléinium, 5-formyl-1-éthylquinoléinium, 6-formyl-1-éthylquinoléinium, 7-formyl-1-éthylquinoléinium, 8-formyl-1-éthylquinoléinium, 5-formyl-1-benzylquinoléinium, 6-formyl-1-benzylquinoléinium, 7-formyl-1-benzylquinoléinium, 8-formyl-1-benzylquinoléinium, 5-formyl-1-allylquinoléinium, 6-formyl-1-allylquinoléinium, 7-formyl-1-allylquinoléinium et 8-formyl-1-allylquinoléinium, 5-acétyl-1-méthylquinoléinium, 6-acétyl-1-méthylquinoléinium, 7-acétyl-1-méthylquinoléinium, 8-acétyl-1-méthylquinoléinium, 5-acétyl-1-éthylquinoléinium, 6-acétyl-1-éthylquinoléinium, 7-acétyl-1-éthylquinoléinium, 8-acétyl-1-éthylquinoléinium, 5-acétyl-1-benzylquinoléinium, 6-acétyl-1-benzylquinoléinium, 7-acétyl-1-benzylquinoléinium, 8-acétyl-1-benzylquinoléinium, 5-acétyl-1-allylquinoléinium, 6-acétyl-1-allylquinoléinium, 7-acétyl-1-allylquinoléinium et 8-acétyl-1-allylquinoléinium, 9-formyl-10-méthylacridinium, 4-(2'-formylvinyl)-1-méthylpyridinium, 1,3-diméthyl-2-(4'-formylphényl)-benzimidazolium, 1,3-diméthyl-2-(4'-formylphényl)-imidazolium, 2-(4'-formylphényl)-3-méthylbenzothiazolium, 2-(4'-acétylphényl)-3-méthylbenzothiazolium, 2-(4'-formylphényl)-3-méthylbenzoxazolium, 2-(5'-formyl-2'-furyl)-3-méthylbenzothiazolium, 2-(5'-formyl-2'-furyl)-3-méthylbenzothiazolium, 2-(5'-formyl-2'-thiényl)-3-méthylbenzothiazolium, 2-(3'-formylphényl)-3-méthylbenzothiazolium, 2-(4'-formyl-1-naphtyl)-3-méthylbenzothiazolium, 5-chloro-2-(4'-formylphényl)-3-méthylbenzothiazolium, 2-(4'-formylphényl)-3,5-diméthylbenzothiazolium l'isatine, la 1-méthylisatine, la 1-allylisatine, la 1-hydroxyméthylisatine, la 5-chloro-isatine, la 5-méthoxyisatine, la 5-nitro-isatine, la 6-nitro-isatine, la 5-sulfo-isatine, la 5-carboxyisatine, la quinisatine, la 1-méthylquinisatine, ainsi que des mélanges quelconques des composés susmentionnés.

7. Agent selon l'une quelconque des revendications 1 à 6, **caractérisé en ce que** le composant B est choisi dans le groupe constitué par la vanilline, le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxybenzaldéhyde, le 4-hydroxy-2,5-diméthoxybenzaldéhyde, le 4-hydroxy-2,6-diméthoxybenzaldéhyde, le 4-hydroxy-2-méthylbenzaldéhyde, le 4-hydroxy-3-méthylbenzaldéhyde, le 4-hydroxy-2,3-diméthylbenzaldéhyde, le 4-hydroxy-2,5-diméthylbenzaldéhyde, le 4-hydroxy-2,6-diméthylbenzaldéhyde, le 4-hydroxy-3,5-diméthoxybenzaldéhyde, le 4-hydroxy-3,5-diméthylbenzaldéhyde, le 3,5-diéthoxy-4-hydroxybenzaldéhyde, le 2,6-diéthoxy-4-hydroxybenzaldéhyde, le 3-hydroxy-4-méthoxybenzaldéhyde, le 2-hydroxy-4-méthoxybenzaldéhyde, le 2-éthoxy-4-hydroxybenzaldéhyde, le 3-éthoxy-4-hydroxybenzaldéhyde, le 4-éthoxy-2-hydroxybenzaldéhyde, le 4-éthoxy-3-hydroxybenzaldéhyde, le 2,3-diméthoxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,5-diméthoxybenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,4-dihydroxy-3-méthylbenzaldéhyde, le 2,4-dihydroxy-5-méthylbenzaldéhyde, le 2,4-dihydroxy-6-méthylbenzaldéhyde, le 2,4-dihydroxy-3-méthoxybenzaldéhyde, le 2,4-dihydroxy-5-méthoxybenzaldéhyde, le 2,4-dihydroxy-6-méthoxybenzaldéhyde, le 2,5-dihydroxybenzaldéhyde, le 2,6-dihydroxybenzaldéhyde, le 3,4-dihydroxybenzaldéhyde, le 3,4-dihydroxy-2-méthylbenzaldéhyde, le 3,4-dihydroxy-5-méthylbenzaldéhyde, le 3,4-dihydroxy-6-méthylbenzaldéhyde, le 3,4-dihydroxy-2-méthoxybenzaldéhyde, le 3,4-dihydroxy-5-méthoxybenzaldéhyde, le 3,5-dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 2,5,6-trihydroxybenzaldéhyde, le 3,4,5-trihydroxybenzaldéhyde, le 4-hydroxy-2-méthoxybenzaldéhyde, le 4-diméthylaminobenzaldéhyde, le 4-diéthylaminobenzaldéhyde, le 4-diméthylamino-2-hydroxybenzaldéhyde, le 4-diéthylamino-2-hydroxybenzaldéhyde, le 4-pyrrolidinobenzaldéhyde, le 4-morpholinobenzaldéhyde, le 2-morpholinobenzaldéhyde, le 4-pipéridinobenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-napthaldéhyde, le 4-hydroxy-3-méthoxy-1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-hydroxy-1-naphtaldéhyde, le 4-diméthylamino-1-naphtaldéhyde, l'aldéhyde 4-diméthylaminocinnamique, le 2-diméthylaminobenzaldéhyde, le 2-chloro-4-diméthylaminobenzaldéhyde, le 4-diméthylamino-2-méthylbenzaldéhyde, l'aldéhyde 4-diéthylaminocinnamique, le 4-dibutylaminobenzaldéhyde, le 4-diphénylaminobenzaldéhyde, le 4-diméthylamino-2-méthoxybenzaldéhyde, le 4-(1-imidazolyl)-benzaldéhyde et le pipéronal.

8. Agent selon l'une quelconque des revendications 1 à 7, **caractérisé en ce qu'**il contient au moins un composé comme composant C, choisi parmi (a) les composés CH-acides et (b) les composés avec un groupe amino primaire ou secondaire ou un groupe hydroxy, choisis parmi les composés hydroxy aromatiques, les amines aromatiques primaires ou secondaires et les composés hétérocycliques contenant de l'azote.

9. Agent selon la revendication 9, **caractérisé en ce que** les composés CH-acides du composant C sont choisis dans le groupe constitué de l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline (base de Fischer), l'iodure de 2,3-diméthylbenzothiazolium, le p-toluènesulfonate de 2,3-diméthylbenzothiazolium, le p-toluènesulfonate de 2,3-diméthylnaphto[1,2-d]thiazolium, le p-toluènesulfonate de 3-éthyl-2-méthylnaphto[1,2-d]thiazolium, la rhodanine, l'acide rhodanine-3-acétique, l'iodure de 1,4-diméthylquinoléinium, l'iodure de 1,2-diméthylquinoléinium, l'acide barbiturique, l'acide thiobarbiturique, l'acide 1,3-diméthylthiobarbiturique, l'acide 1,3-diéthylthiobarbiturique, l'acide 1,3-diéthylbarbiturique, l'oxindole, l'acétate de 3-indoxyle, la 2-coumaranone, la 5-hydroxy-2-coumaranone, la 6-hydroxy-2-coumaranone, la 3-méthyl-1-phénylpyrazolin-5-one, l'indane-1,2-dione, l'indane-1,3-dione, l'indan-4-one, le benzoylacétonitrile, la 3-dicyanométhylène-indan-1-one, le chlorhydrate de 2-amino-4-imino-1,3-thiazoline, la 5,5-diméthylcyclohexane-1,3-dione, la 2H-1,4-benzoxazin-4H-3-one, l'iodure de 3-éthyl-2-méthylbenzoxazolium, l'iodure de 3-éthyl-2-méthylbenzothiazolium, l'iodure de 1-éthyl-4-méthylquinoléinium, l'iodure de 1-éthyl-2-méthylquinoléinium, l'iodure de 1,2,3-triméthylquinoxalinium, le p-toluènesulfonate de 3-éthyl-2-méthylbenzoxazolium, le p-toluènesulfonate de 3-éthyl-2-méthylbenzothiazolium, le p-toluènesulfonate de 1-éthyl-4-méthylquinoléinium, le p-toluènesulfonate de 1-éthyl-2-méthylquinolinium, le p-toluènesulfonate de 1,2,3-triméthylquinoxalinium, le chlorure de 1,2-dihydro-1,3,4,6-tétraméthyl-2-oxopyrimidinium, l'hydrogénosulfate de 1,2-dihydro-1,3,4,6-tétraméthyl-2-oxopyrimidinium, le chlorure de 1,2-dihydro-1,3,4-triméthyl-2-oxopyrimidinium, le chlorure de 1,2-dihydro-4,6-diméthyl-1,3-dipropyl-2-oxopyrimidinium, l'hydrogénosulfate de 1,2-dihydro-1,3,4,6-tétraméthyl-2-thioxopyrimidinium et le chlorure de 2-dihydro-1,3,4,5,6-pentaméthyl-2-oxopyrimidinium.

10. Agent selon la revendication 8, **caractérisé en ce que** les amines aromatiques primaires et secondaires du composant C sont choisies dans le groupe constitué de la N,N-diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morpholinoaniline, le 2-aminophénol, le 3-aminophénol, le 4-aminophénol, le 2-aminométhyl-4-aminophénol, le 2-hydroxyméthyl-4-aminophénol, l'o-phénylènediamine, le m-phénylènediamine, le p-phénylènediamine, le 2,5-diaminotoluène, le 2,5-diaminophénol, le 2,5-diaminoanisol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, le 3-amino-4-(2-hydroxyéthyloxy)phénol, le 3,4-méthylènedioxyphénol, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxyéthylamino)-4-méthoxy-2-méthylphénol, le 4-amino-2-hydroxyméthylphénol, le 2-(diéthylaminométhyl)-4-aminophénol, le 4-amino-1-hydroxy-2-(2-hydroxyéthylaminométhyl)-benzène, le 1-hydroxy-2-amino-5-méthylbenzène, le 1-hydroxy-2-amino-6-méthylbenzène, le 2-amino-5-acétamidophénol, le 1,3-diméthyl-2,5-diaminobenzène, le 5-(3-hydroxypropylamino-)2-méthylphénol, le 5-amino-4-méthoxy-2-méthylphénol, le N,N-diméthyl-3-aminophénol, le N-cyclopentyl-3-aminophénol, le 5-amino-4-fluoro-2-méthylphénol, le 2,4-diamino-5-fluorotoluène, le 2,4-diamino-5-(2-hydroxyéthoxy)-toluène, le 2,4-diamino-5-méthylphénétol, le 3,5-diamino-2-méthoxy-1-méthylbenzène, le 2-amino-4-(2-hydroxyéthylamino)-anisol, le 2,6-bis-(2-hydroxyéthylamino)-1-méthylbenzène, le 1,3-diamino-2,4-diméthoxybenzène, le 3,5-diamino-2-méthoxytoluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxybenzoïque, l'acide 4-amino-3-hydroxybenzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphténique, l'acide 3-aminophtalique, l'acide 5-amino-isophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, la 4,5-diaminopyrocatéchine, le 4,6-diaminopyrogallol, la 1-(2-hydroxy-5-aminobenzyl)-2-imidazolidinone, le 4-amino-2-((4-[(5-amino-2-hydroxyphényl)méthyl]-pipérazinyl)méthyl)phénol, la 3,5-diamino-4-hydroxypyrocatéchine, le 1,4-bis-(4-aminophényl)-1,4-diazacycloheptane, les nitriles aromatiques tels que le 2-amino-4-hydroxybenzonitrile, le 4-amino-2-hydroxybenzonitrile, le 4-aminobenzonitrile, le 2,4-diaminobenzonitrile, les composés amino contenant des groupes nitro, tels que la 3-amino-6-méthylamino-2-nitropyridine, l'acide picramique, le chlorure de [8-[(4-amino-2-nitrophényl)-azo]-7-hydroxynapht-2-yl]-triméthylammonium, le chlorure de [8-((4-amino-3-nitrophényl)-azo)-7-hydroxynapht-2-yl]-triméthylammonium (Basic Brown 17), le 1-hydroxy-2-amino-4,6-dinitrobenzène, le 1-amino-2-nitro-4-(bis-(2-hydroxyéthyl)amino]-benzène, le 1-amino-2-[(2-hydroxyéthyl)amino]-5-nitrobenzène (HC Yellow n° 5), le 1-amino-2-nitro-4-[(2-hydroxyéthyl)amino]-benzène (HC Red n° 7), la 2-chloro-5-nitro-N-2-hydroxyéthyl-1,4-phénylènediamine, le 1[(2-hydroxyéthyl)amino]-2-nitro-4-aminobenzène (HC Red n° 3), le 4-amino-3-nitrophénol, le 4-amino-2-nitrophénol, la 6-nitro-o-toluidine, le 1-amino-3-méthyl-4-[(2-hydroxyéthyl)amino]-6-nitrobenzène (HC Violet n° 1), le 1-amino-2-nitro-4-[(2,3-dihydroxypropyl)amino]-5-chlorobenzène (HC Red n° 10), l'acide 2-(4-amino-2-nitroanilino)-benzoïque, la 6-nitro-2,5-diaminopyridine, le 2-amino-6-chloro-4-nitrophénol, le sel disodique de l'acide 1-amino-2-(3-nitrophénylazo)-7-phénylazo-6-naphtol-3,6-disulfonique (Acid blue n° 29), le sel disodique de l'acide 1-amino-2-(2-hydroxy-4-nitrophénylazo)-8-naphtol-3,6-disulfonique (Palatinchrome green), le sel disodique de l'acide 1-amino-2-(3-chloro-2-hydroxy-5-nitrophénylazo)-8-naphtol-3,6-disulfonique (Gallion), le sel disodique de l'acide 4-amino-4'-nitrostilbène-2,2'-disulfonique, le 2,4-diamino-3',5'-dinitro-2'-hydroxy-5-méthylazobenzène (Mordant brown 4), l'acide 4'-amino-4-nitrodiphénylamine-2-sulfonique, l'acide 4'-amino-3'-nitrobenzophénone-2-carboxylique, le 1-amino-4-nitro-2-(2-nitrobenzylidèneamino)-benzène, la 2-[2-(diéthylamino)éthylamino]-5-nitroaniline, l'acide 3-amino-4-hydroxy-5-nitrobenzènesulfonique, le 3-amino-3'-nitrobiphényle, le 3-amino-4-nitroacénaphtène, le 2-amino-1-nitronaphtalène, le 5-amino-6-nitrobenzo-1,3-dioxol, les anilines, en particulier les anilines contenant des groupes nitro, tels que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)-benzène, le 2-nitro-1-amino-4-(bis-(2-hydroxyéthyl)-amino]-benzène, l'acide 4-amino-2-nitrodiphénylamine-2'-carboxylique, le 1-amino-5-chloro-4-(2-hydroxyéthylamino)-2-nitrobenzène, les anilines aromatiques ou, selon le cas, les phénols présentant un autre radical aromatique, tels qu'ils sont représentés dans la formule VII 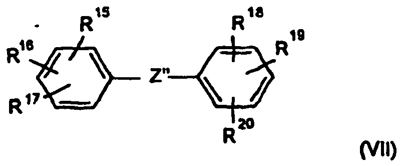 où
R¹⁵ représente un groupe hydroxy ou amino, qui peut être substitué par des groupes C₁-C₆-alkyle, C₁-C₆-hydroxyalkyle, C₁-C₆-alcoxy- ou C₁-C₆-alcoxy-C₁-C₆-alkyle,
R¹⁶, R¹⁷, R¹⁸, R¹⁹ et R²⁰ représentent, indépendamment l'un de l'autre, un atome d'hydrogène, un groupe hydroxy ou amino, qui peut être substitué par des groupes C₁-C₆-alkyle, C₁-C₆-hydroxyalkyle, C₁-C₆-alcoxy-, C₁-C₆-aminoalkyle ou C₁-C₆-alcoxy-C₁-C₆-alkyle, et
Z" représente une liaison directe, une chaîne carbonée comprenant 1 à 4 atomes de carbone, saturée ou insaturée, le cas échéant substituée par des groupes hydroxy, un groupe carbonyle, sulfonyle ou imino, un atome d'oxygène ou de soufre ou un groupe de formule VIII
-Q'(_{̅}-CH₂-Q-CH₂-Q")_{̅o} (VIII)
où
Q signifie une liaison directe, un groupe CH₂ ou CHOH,
Q' et Q" représentent, indépendamment l'un de l'autre, un atome d'oxygène, un groupe NR²¹, où R²¹ signifie un atome d'hydrogène, un groupe C₁-C₆-alkyle ou un groupe hydroxy-C₁-C₆-alkyle, les deux groupes pouvant également former, ensemble avec le reste de la molécule, un cycle de 5, 6 ou 7 chaînons, le groupe O-(CH₂)ₚ-NH ou NH-(CH₂)_{p'}-O, où p et p' représentent 2 ou 3, et
o signifie un nombre de 1 à 4,
tels qu'en particulier le 4,4'-diaminostilbène et son chlorhydrate, le sel monosodique ou disodique de l'acide 4,4'-diaminostilbène-2,2'-disulfonique, le 4-amino-4'-diméthylaminostilbène et son chlorhydrate, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diaminobenzophénone, le 4,4'-diaminodiphényléther, le 3,3',4,4'-tétraaminodiphényle, la 3,3',4,4'-tétraaminobenzophénone, le 1,3-bis-(2,4-diaminophénoxy)-propane, le 1,8-bis-(2,5-diaminophénoxy)-3,6-dioxaoctane, le 1,3-bis-(4-aminophénylamino)propane, le 1,3-bis-(4-aminophénylamino)-2-propanol, le 1,3-bis-[N-(4-aminophényl)-2-hydroxyéthylamino]-2-propanol, la N,N-bis-[2-(4-aminophénoxy)-éthyl]-méthylamine, la N-phényl-1,4-phénylènediamine et le bis-(5-amino-2-hydroxyphényl)-méthane.

11. Agent selon la revendication 8, **caractérisé en ce que** les composés hydroxy aromatiques du composé C sont choisis dans le groupe constitué de 2-méthylrésorcinol, le 4-méthylrésorcinol, le 5-méthylrésorcinol, le 2,5-diméthylrésorcinol, le résorcinol, le 3-méthoxyphénol, la pyrocatéchine, l'hydroquinone, le pyrogallol, la phloroglucine, l'hydroxyhydroquinone, le 2-méthoxyphénol, le 3-méthoxyphénol, le 4-méthoxyphénol, le 3-diméthylaminophénol, le 2-(2-hydroxyéthyl)phénol, le 3,4-méthylènedioxyphénol, l'acide 2,4-dihydroxybenzoïque, l'acide 3,4-dihydroxybenzoïque, l'acide 1-(2,4-dihydroxyphényl)acétique, l'acide 1-(3,4-dihydroxyphényl)acétique, l'acide biliaire, l'acide 2,4,6-trihydroxybenzoïque, la 2,4,6-trihydroxyacétophénone, le 2-chlororésorcinol, le 4-chlororésorcinol, le 1-naphtol, le 1,5-dihydroxynaphtalène, le 2,3-dihydroxynaphtalène, le 2,7-dihydroxynaphtalène, l'acide 6-diméthylamino-4-hydroxy-2-naphtalènesulfonique et l'acide 3,6-dihydroxy-2,7-naphtalènesulfonique.

12. Agent selon la revendication 8, **caractérisé en ce que** les composés hétérocycliques contenant de l'azote du composé C sont choisis dans le groupe constitué de la 2-aminopyridine, la 3-aminopyridine, la 4-aminopyridine, la 2-amino-3-hydroxypyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophényl)aminoéthyl]-N-(5-amino-2-pyridyl)-amine, la N-[2-(4-aminophényl)aminoéthyl]-N-(5-amino-2-pyridyl)-amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétraaminopyrimidine, la 2-méthylamino-4,5,6-triaminopyrimidine, la 2,4-diaminopyrimidine, la 4,5-diaminopyrimidine, la 2-amino-4-méthoxy-6-méthylpyrimidine, le 3,5-diaminopyrazole, le 3,5-diamino-1,2,4-triazole, le 3-aminopyrazole, le 3-amino-5-hydroxypyrazole, le 1-phényl-4,5-diaminopyrazole, le 1-(2-hydroxyéthyl)-4,5-diaminopyrazole, le 1-phényl-3-méthyl-4,5-diaminopyrazole, la 4-amino-2,3-diméthyl-1-phényl-3-pyrazolin-5-one (4-aminoantipyrine), la 1-phényl-3-méthylpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-amino-isoquinoléine, le 5-amino-indazole, le 6-amino-indazole, le 5-aminobenzimidazole, le 7-aminobenzimidazole, le 5-aminobenzothiazole, le 7-aminobenzothiazole, la 2,5-dihydroxy-4-morpholinoaniline ainsi que les dérivés d'indole et d'indoline, tels que le 4-amino-indole, le 5-amino-indole, le 6-amino-indole, le 7-amino-indole, le 5,6-dihydroxyindole, la 5,6-dihydroxyindoline, la 4-hydroxyindoline et les dérivés d'hydroxypyrimidine et les sels physiologiquement acceptables des composés susmentionnés.

13. Agent selon l'une quelconque des revendications 1 à 12, **caractérisé en ce que** les composés de formule I, les composés du composant B et les composés du composant C sont à chaque fois contenus en une quantité de 0,03 à 65 mmoles, en particulier de 1 à 40 mmoles, par rapport à 100 g de la totalité de l'agent de teinture.

14. Agent selon l'une quelconque des revendications 1 à 13, **caractérisé en ce qu'**il contient au moins un produit de réaction PR selon la formule IX, 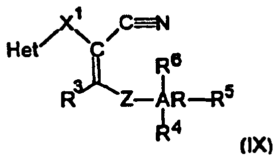 où Het, X¹, R³, R⁴, R⁵, R⁶, AR et Z sont définis comme dans la revendication 5, à condition que lorsque R³ représente un atome d'hydrogène et Z représente une liaison directe et le groupement -[ArR⁴R⁵R⁶] de formule IX représente un groupe 3,5-dichloro-4-hydroxyphényle, Het ne soit pas dérivé de benzofuranne, de thiazole ou de benzothiazole.

15. Agent selon la revendication 14, **caractérisé en ce que** AR selon la formule IX représente du benzène ou du naphtalène.

16. Agent selon l'une quelconque des revendications 14 à 15, **caractérisé en ce que** Z selon la formule IX représente une liaison directe ou un groupe vinylène.

17. Agent selon l'une quelconque des revendications 14 à 16, **caractérisé en ce que** R³ selon la formule IX représente un atome d'hydrogène.

18. Agent selon l'une quelconque des revendications 14 à 17, **caractérisé en ce que** le produit de réaction PR est contenu en une quantité de 0,03 à 65 mmoles, en particulier de 1 à 40 mmoles, par rapport à 100 g de l'agent de teinture dans sa totalité.

19. Agent selon l'une quelconque des revendications 1 à 18, **caractérisé en ce qu'**il contient un agent de renforcement de la couleur choisi dans le groupe constitué de la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'arginine, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

20. Agent selon l'une quelconque des revendications 1 à 19, **caractérisé en ce qu'**il contient au moins un colorant montant directement sur la fibre, de préférence en une quantité de 0,01 à 20% en poids, par rapport à la totalité de l'agent de teinture.

21. Agent selon l'une quelconque des revendications 1 à 20, **caractérisé en ce qu'**il contient, comme précurseur d'un colorant par oxydation, au moins un composant de développement et le cas échéant au moins un composant de couplage.

22. Agent selon l'une quelconque des revendications 1 à 21, **caractérisé en ce qu'**on ajoute des sels d'ammonium ou de métal, choisis dans le groupe formé par les formiates, les carbonates, les halogénures, les sulfates, les butyrates, les valériates, les capronates, les acétates, les lactates, les glycolates, les tartrates, les citrates, les gluconates, les propionates, les phosphates et les phosphonates de métaux alcalins, tels que le potassium, le sodium, ou le lithium, de métaux alcalino-terreux, tels que le magnésium, le calcium, le strontium ou le baryum, ou d'aluminium, de manganèse, de fer, de cobalt, de cuivre ou de zinc.

23. Agent selon l'une quelconque des revendications 1 à 22, **caractérisé en ce qu'**il contient en outre des oxydants, en particulier H₂O₂ en une quantité de 0,01 à 6% en poids, par rapport à la solution d'utilisation.

24. Agent selon l'une quelconque des revendications 1 à 23, **caractérisé en ce qu'**il contient en outre des agents tensioactifs anioniques, zwittérioniques ou non ioniques.

25. Utilisation d'au moins un composé selon la formule I selon la revendication 1, en combinaison avec au moins un composé carbonyle réactif, comme composant de teinture dans des agents de teinture pour cheveux.

26. Procédé pour la teinture de fibres kératiniques, en particulier de cheveux, dans lequel un agent de teinture selon la revendication 1, ainsi que des constituants cosmétiques usuels sont appliqués sur les fibres kératiniques, on les laisse pendant un certain laps de temps, usuellement d'environ 15-30 minutes sur les fibres, puis on les élimine par rinçage ou par lavage avec un shampooing.

27. Procédé selon la revendication 26, **caractérisé en ce qu'**on applique, dans un procédé à deux étapes, le composant B avant ou après l'application du composé selon la formule I sur les fibres kératiniques, on laisse le mélange obtenu sur les cheveux pendant un certain laps de temps, usuellement d'environ 15-30 minutes, sur les fibres, puis on l'élimine par rinçage ou par lavage avec un shampooing.

28. Procédé selon l'une quelconque des revendications 26 ou 27, **caractérisé en ce que** les fibres kératiniques, avant l'utilisation d'un agent de teinture selon la revendication 1, dans le cadre d'un prétraitement, sont éclaircies avec un agent de décoloration ou teintes avec un agent de teinture par oxydation.

29. Utilisation d'au moins un composé selon la formule I, 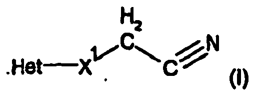 Het et X¹ étant définis comme dans la revendication 1, en combinaison avec des composés carbonyle réactifs (composant B), pour nuancer des teintures par oxydation de fibres kératiniques, en particulier de cheveux.

30. Utilisation d'au moins un composé selon la formule I, 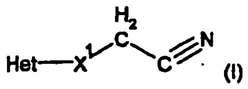 Het et X¹ étant définis comme dans la revendication 1, en combinaison avec des composés carbonyle réactifs (composant B), pour rafraîchir la couleur de fibres kératiniques teintées avec des agents de teinture par oxydation.
